(19) Europäisches Patentamt
European Patent Office
Office européen des brevets

(11) **EP 2 063 855 B1**

(12) **EUROPEAN PATENT SPECIFICATION**

(45) Date of publication and mention
of the grant of the patent:
**03.04.2013 Bulletin 2013/14**

(21) Application number: **07807586.8**

(22) Date of filing: **12.09.2007**

(51) Int Cl.:
*A61K 8/27* (2006.01)     *A61K 8/19* (2006.01)
*A61K 8/28* (2006.01)     *A61K 8/29* (2006.01)
*A61K 8/81* (2006.01)     *A61Q 17/04* (2006.01)
*A61K 8/04* (2006.01)     *C09C 3/10* (2006.01)

(86) International application number:
**PCT/JP2007/068218**

(87) International publication number:
**WO 2008/032859 (20.03.2008 Gazette 2008/12)**

(54) **ULTRAVIOLET SCREENING AGENT FOR COSMETICS AND COSMETICS USING THE SAME**

ULTRAVIOLETT-SCHUTZMITTEL FÜR KOSMETIKA UND KOSMETISCHE VERWENDUNG DES MITTELS

AGENT FILTRANT LES ULTRAVIOLETS POUR DES PRODUITS COSMÉTIQUES ET PRODUITS COSMÉTIQUES UTILISANT CELUI-CI

(84) Designated Contracting States:
**DE FR**

(30) Priority: **15.09.2006 JP 2006251783**
**29.03.2007 JP 2007088942**
**03.09.2007 JP 2007227912**

(43) Date of publication of application:
**03.06.2009 Bulletin 2009/23**

(73) Proprietors:
• **Nippon Shokubai Co., Ltd.**
**Osaka-shi, Osaka 541-0043 (JP)**
• **Tayca Corporation**
**Osaka-shi, Osaka 551-0022 (JP)**

(72) Inventors:
• **MATSUMOTO, Takeshi**
**Takatsuki-shi**
**Osaka 569-0854 (JP)**
• **OKA, Hideo**
**Osaka-shi**
**Osaka 551-0022 (JP)**

• **YAMASHITA, Jun**
**Osaka-shi**
**Osaka 551-0022 (JP)**

(74) Representative: **Perrey, Ralf et al**
**Müller-Boré & Partner**
**Grafinger Strasse 2**
**81671 München (DE)**

(56) References cited:
EP-A1- 1 167 462      EP-A1- 1 405 624
EP-A1- 1 530 964      WO-A1-00/42112
WO-A1-01/93812      DE-A1-102004 041 536
DE-A1-102005 010 803      JP-A- 08 337 514
JP-A- 11 302 015      JP-A- 2005 145 972
JP-A- 2007 277 415      US-A- 3 897 586
US-B1- 6 464 965

Remarks:
The file contains technical information submitted after the application was filed and not included in this specification

**Description**

The present invention relates to an ultraviolet screening agent for cosmetics and cosmetics using the same.

Zinc oxide and titanium oxide have excellent ultraviolet screening ability, and therefore, they are expected to be used as an ultraviolet screening agent for cosmetics. These metal oxides have high refractive indexes and high opacifying properties, so that they are liable to unnaturally whiten on the skin. When transparency suitable for cosmetics is tried to be secured, the metal oxides need to be dispersed in the form of fine particles having a particle diameter of 100 nm or smaller. However, when the particle diameter becomes small, the surface area is increased, so that it becomes difficult to maintain a disperse state. In addition, zinc oxide is an amphoteric oxide, and therefore, zinc oxide dissolves in water under acidic or basic conditions and, for example, causes allergic symptoms on the skin in some cases. Titanium oxide is a strong photocatalyst, and therefore, it sometimes deteriorates other formulation ingredients. Further, in general, metal oxides are inferior in a feel to the skin, compared to organic compounds, and therefore, the metal oxides have a tendency not to be favorably added to cosmetics.

Thus, for example, in Japanese Patent Laid-Open (Kokai) Publication No. Hei 3-183620, Japanese Patent Laid-Open (Kokai) Publication No. Hei 11-302015, and Japanese Patent Laid-Open (Kokai) Publication No. 2001-58821, there have been various attempts to solve the above-described problems by coating the surface of each of zinc oxide fine particles with silica or the like to suppress surface activity while maintaining ultraviolet screening ability. However, if the surface of each of zinc oxide fine particles is only treated with an inorganic compound, the treated zinc oxide is not still satisfiable, particularly with regard to dispersibility and a feel to the skin, when it is added to cosmetics.

For the purpose of enhancing the stability of the forms of water-based cosmetics, a thickening polymer having carboxyl groups on side chains has usually been used as a thickener. Examples of the thickening polymer having carboxyl groups on side chains may include carboxyvinyl polymer, carboxymethyl cellulose, carboxymethyl starch, sodium polyacrylate, propylene glycol esters of alginic acid, and alginic acid salts. In these thickening polymers, carboxyvinyl polymer has frequently been used. Carboxyvinyl polymer has widely been used for cosmetics, because this polymer gives high thickening stability at low concentrations and reproducibility which cannot be obtained by natural macromolecules, and have excellent temperature stability and excellent resistance to bacteria, and can be used in wide ranges of pH and viscosity, and have an excellent feel of use when it is applied to the skin, as characteristics and advantages thereof.

However, when zinc oxide fine particles or titanium oxide fine particles are added to cosmetics using a carboxyvinyl polymer, there are serious problems that the deterioration of thickening properties and the aggregation of fine particles may occur. In order to solve these problems, Domestic Re-publication of PCT International Publication for Patent Application WO 01/093812 proposes a method in which the surface of each of fine particles is treated with silica or alumina. However, the performance of the resulting silica-coated or alumina-coated fine particles is not sufficient.

DE 10 2004 041 536 A1 discloses the use of random copolymers as dispersants for the preparation of dispersions having incompatible disperse and continuous phases, in particular for the dispersal of particles having a hydrophilic surface in oils, and dispersions or powder compositions comprising random copolymers and particles having a hydrophilic surface, and processes for the preparation thereof. DE 10 2005 010 803 A1 discloses cosmetic and dermatological preparations for protecting human skin and hair against UV radiation, comprising surface-coated titanium dioxide particles which are transparent in the visible region and have a crystallite size of from 10 to 20 nm and a specific surface area of from 90 to 110 $m^2$/g, wherein the surface coating of the titanium dioxide particles comprises a multicoating of a) aluminum oxide and b) methicone or a copolymer of methicone and dimethicone or a multicoating of a) aluminum oxide, b) methicone or a copolymer of methicone and dimethicone and c) silicon dioxide.

Under the above-described circumstances, it is an object of the present invention to provide an ultraviolet screening agent for cosmetics, which has excellent dispersibility and excellent storage stability while maintaining high transparency and excellent ultraviolet screening ability and which gives cosmetics exhibiting a remarkably improved feel to the skin when added to the cosmetics; and further provides cosmetics using the same.

The present inventors have made various studies, and as a result, they have found that the above object can be attained when polymer-coated metal oxide fine particles produced by coating, with silica as a first layer, the surface of each of specific metal oxide fine particles, a primary particle diameter of which fine particles is not smaller than 1 nm and not greater than 100 nm, and coating the outside of the first layer with a specific polymer as a second layer, are added to an ultraviolet screening agent, thereby completing the present invention.

That is, the present invention provides an ultraviolet screening agent for cosmetics, comprising polymer-coated metal oxide fine particles produced by coating, with silica as a first layer, the surface of each of at least one kind of metal oxide fine particles selected from the group consisting of zinc oxide fine particles, titanium oxide fine particles, cerium oxide fine particles, zirconium oxide fine particles, and iron oxide fine particles, a primary particle diameter of which fine particles is not smaller than 1 nm and not greater than 100 nm, and coating the outside of the first layer with a polymer as a second layer, wherein the polymer coating is chemically bonded to the surface of each of the silice-coated metal oxide fine particles and wherein the polymer includes (meth)acrylic-type polymers, styrene-type polymers, vinyl acetate-type polymers, vinyl chloride-type polymers, vinylidene-type polymers, and their copolymers used alone or two or more kinds of these polymers may be used in combination.

In the ultraviolet screening agent for cosmetics according to the present invention, the polymer-coated metal oxide fine particles are, for example, in the form of a dispersion and/or a powder.

The present invention further provides a cosmetic comprising the above ultraviolet screening agent for cosmetics. The cosmetic may preferably further comprise a carboxyvinyl polymer as a hydrophilic thicker or an emulsion stabilizer. The term "carboxyvinyl polymer" as used herein means a hydrophilic sticker or an emulsion stabilizer also called "carbomer" in the fields of cosmetics and cosmetic bases, which carbomer is an acrylic polymer cross-linked with an allyl ether of pentaerythritol, an allyl ether of sucrose, or an allyl ether of propylene.

According to the present invention, the use of a dispersion and/or a powder of specific polymer-coated metal oxide fine particles makes it possible to obtain an ultraviolet screening agent for cosmetics, which has excellent dispersibility and excellent storage stability while maintaining high transparency and excellent ultraviolet screening ability and which gives cosmetics exhibiting a remarkably improved feel to the skin when added to the cosmetics; and further obtain such excellent cosmetics.

«Ultraviolet screening agent for cosmetics»

The ultraviolet screening agent for cosmetics according to the present invention comprises polymer-coated metal oxide fine particles produced by coating, with silica as a first layer, the surface of at least one kind of metal oxide fine particles selected from the group consisting of zinc oxide fine particles, titanium oxide fine particles, cerium oxide fine particles, zirconium oxide fine particles, and iron oxide fine particles, a primary particle diameter of which fine particles is not smaller than 1 nm and not greater than 100 nm, and coating the outside of the first layer with a polymer as the second layer wherein the polymer coating is chemically bonded to the surface of each of the silica-coated metal oxide fine particles and wherein the polymer includes (meth)acrylic-type polymers, styrene-type polymers, vinyl acetate-type polymers, vinyl chloride-type polymers, vinylidene-type polymers, and their copolymers used alone or two or more kinds of these polymers may be used in combination. The term "primary particle diameter" as used herein means the particle diameter of primary particles as minimum particles constituting metal oxide fine particles, and in the present invention, it is a number-average particle diameter evaluated by an image analysis method as described below in Examples.

In the ultraviolet screening agent for cosmetics according to the present invention, the polymer-coated metal oxide fine particles are, for example, in the form of a dispersion and/or a powder.

In the ultraviolet screening agent for cosmetics according to the present invention, the primary particle diameter of each of the metal oxide fine particles is not smaller than 1 nm and not greater than 100 nm, preferably not smaller than 3 nm and not greater than 80 nm, and more preferably not smaller than 5 nm and not greater than 50 nm. When the primary particle diameter of each of the metal oxide fine particles is smaller than 1 nm, the metal oxide fine particles readily aggregate and become difficult to handle in some cases. In contrast, when the primary particle diameter of each of the metal oxide fine particles is greater than 100 nm, the surface of each of the metal oxide fine particles cannot uniformly be coated in some cases, when the surface of each of the metal oxide fine particles is coated with silica as the first layer, or when the outside of the first layer is coated with a polymer as the second layer.

In the polymer-coated metal oxide fine particles, the thickness of the silica layer as the first layer may preferably be not smaller than 1 nm and not greater than 100 nm, more preferably not smaller than 2 nm and not greater than 60 nm, and still more preferably not smaller than 3 nm and not greater than 40 nm. When the thickness of the silica layer is smaller than 1 nm, the metal oxide fine particles cannot sufficiently be coated with silica in some cases. In contrast, the thickness of the silica layer is greater than 100 nm, a transparent feel when a cosmetic containing the polymer-coated metal oxide fine particles is applied to the skin is decreased in some cases. In addition, the thickness of the silica layer mean the thickness of the thickest portion of the silica layer formed as the first layer on the surface of each of the metal oxide fine particles, and is a value evaluated from an image analysis method using an electron microscope.

In the polymer-coated metal oxide fine particles, the thickness of the polymer layer as the second layer may preferably be not smaller than 1 nm and not greater than 100 nm, more preferably not smaller than 2 nm and not greater than 80 nm, and still more preferably not smaller than 3 nm and not greater than 60 nm. When the thickness of the polymer layer is smaller than 1 nm, the dispersability and storage stability of the polymer-coated metal oxide fine particles are not improved in some cases. In contrast, when the thickness of the polymer layer is greater than 100 nm, the ultraviolet screening ability of the polymer-coated metal oxide fine particles becomes insufficient, thereby making impossible the effective screening of ultraviolet rays in some cases, or a transparent feel when a cosmetic containing the polymer-coated metal oxide fine particles is applied to the skin is decreased in some cases. In addition, the thickness of the polymer layer means the thickness of the thickest portion of the polymer layer formed as the second layer outside of each of the metal oxide fine particles, and is a value evaluated from an image analysis method using an electron microscope.

The number-average particle diameter of the polymer-coated metal oxide fine particles may preferably be not smaller than 5 nm and not greater than 500 nm, more preferably not smaller than 10 nm and not greater than 300 nm, and still more preferably not smaller than 20 nm and not greater than 200 nm. When the number-average particle diameter of the polymer-coated metal oxide fine particles is smaller than 5 nm, the polymer-coated metal oxide fine particles readily aggregate and become difficult to handle in some cases. In contrast, the number-average particle diameter of the polymer-coated metal oxide fine particles is greater than 500 nm, a transparent feel when a cosmetic containing the polymer-coated metal oxide fine particles is applied to the skin is decreased in some cases.

In the present invention, the number-average particle diameter of the polymer-coated metal oxide fine particles is a value measured by the method described below in Examples, but the "primary particle diameter" has the meaning similarly defined as the case of the metal oxide fine particles, unless otherwise noted. However, the polymer-coated metal oxide fine particles of the present invention may include a case where each of primary particles (i.e., single particles) of the silica-coated metal oxide fine particles is coated with a polymer and a case where each of secondary particles (i.e., fine particle groups in which two or more single particles are aggregated) of the silica-coated metal oxide fine particles is coated with a polymer; and both the polymer-coated metal oxide fine particles are primary particles. Also, the silica-coated metal oxide fine particles of the present invention may include a case where each of the primary particles (i.e., single particles) of the metal oxide fine particles is coated with silica and a case where each of the secondary particles (i.e., fine particle groups in which two or more single particles are aggregated) of the metal oxide fine particles is coated with silica; and both of the silica-coated metal oxide fine particles are primary particles.

The ultraviolet screening agent for cosmetics according to the present invention contains polymer-coated metal oxide fine particles. In each case, the amount, as a solid content, of the polymer-coated metal oxide fine particles to be added may preferably be not smaller than 1% by mass and not greater than 50% by mass, more preferably not smaller than 3% by mass and not greater than 45% by mass, and still more preferably not smaller than 5% by mass and not greater than 40% by mass, relative to the total mass of the ultraviolet screening agent for cosmetics. When the amount of the polymer-coated metal oxide fine particles to be added is smaller than 1% by mass, the ultraviolet screening ability of an ultraviolet screening agent for cosmetics containing the polymer-coated metal oxide fine particles becomes insufficient, thereby making impossible the effective screening of ultraviolet rays in some cases. In contrast, when the amount of the polymer-coated metal oxide fine particles to be added is greater than 50% by mass, the polymer-coated metal oxide fine particles are aggregated to form a high-order structure, so that the dispersibility and storage stability of an ultraviolet screening agent for cosmetics containing the polymer-coated metal oxide fine particles are decreased in some cases.

When the polymer-coated metal oxide fine particles are in the form of a dispersion, the content of the polymer-coated metal oxide fine particles in the dispersion may preferably be not smaller than 1% by mass and not greater than 80% by mass, more preferably not smaller than 5% by mass and not greater than 70% by mass, and still more preferably not greater than 10% by mass and not greater than 60% by mass, relative to the total mass of the dispersion. When the content of the polymer-coated metal oxide fine particles is smaller than 1% by mass, a dispersion medium is used more than necessary, thereby increasing production cost in some cases. In contrast, the content of the polymer-coated metal oxide fine particles is greater than 80% by mass, the polymer-coated metal oxide fine particles are aggregated to form a high-order structure, so that the dispersibility and storage stability of a dispersion containing the polymer-coated metal oxide fine particles are decreased in some cases.

When the polymer-coated metal oxide fine particles is in the form of a dispersion, examples of the dispersion medium may include water; lower alcohols such as ethanol and isopropanol; glycols such as ethylene glycol, propylene glycol, butylene-glycol, polyethylene glycol (having an average molecular weight of from 200 to 2,000); polyhydric alcohols such as polyoxyethylenemethyl glucoside, glycerin, and diglycerin; dimethyl silicones such methyl polysiloxane, octamethyl trisiloxane, and decamethyl tetrasiloxane; cyclic dimethyl silicones such as decamethyl cyclopentasiloxane and octamethyl cyclotetrasiloxane; and methyl phenyl silicones such as methyl phenyl polysiloxane. These dispersion mediums may be used alone, or two or more kinds of these mediums may also be used in combination.

The ultraviolet screening agent for cosmetics according to the present invention can also contain, besides the polymer-coated metal oxide fine particles, various cosmetic bases, which are used in ordinary cosmetics, in a range without damaging functions such as ultraviolet screening ability. Examples of such cosmetic bases may include thickeners, emulsion stabilizers, surfactants, pH regulators, antiseptic agents, antioxidants, and organic ultraviolet absorbers.

The form of the ultraviolet screening agent for cosmetics according to the present invention may appropriately be selected depending on the kinds of cosmetics containing it, and is not particularly limited but may include aqueous dispersions, oil-in-water type (O/W type) dispersions, water-in-oil type (W/O type) dispersions, and multi-phase type (W/O/W type or O/W/O type) dispersions.

In general, in the cases of dispersions such as aqueous dispersions, oil-in-water type (O/W type) dispersions, and multi-phase type (W/O/W type) dispersions, for example, silica-coated titanium oxide fine particles or silica-coated zinc oxide fine particles each have the surface coated with silica as a first layer. Therefore, these silica-coated metal oxide fine particles are stably dispersed in the dispersion medium, when the dispersion is alkaline. However, these silica-coated metal oxide fine particles may have decreased dispersability, so that they may aggregate and precipitate, when the dispersion becomes weakly acidic which is needed for cosmetics and cosmetic bases, for example, when a pH regulator is added thereto. In addition, for the silica-coated zinc oxide fine particles, zinc oxide which is an amphoteric metal oxide may be eluted when the pH changes from neutrality to alkalinity or acidity. However, when the outside of each of these silica-coated metal oxide fine particles is coated with a polymer as a second layer, the resulting fine particles come to stably disperse even at weak acidity and the elution of zinc oxide hardly occurs.

On the other hand, in the cases of dispersions such as water-in-oil type (W/O type) dispersions and multi-phase type (O/W/O type) dispersions, silica-coated metal oxide fine particles may have poor dispersibility, so that they may readily aggregate and precipitate. Also, for example, when a moisturizing agent is added to cosmetics, silica-coated zinc oxide fine particles may adsorb water, so that zinc oxide which is an amphoteric metal oxide may be eluted. However, when the outside of each of these silica-coated metal oxide fine particles is coated with a polymer, the resulting fine particles come to stably disperse and the elution of zinc oxide hardly occurs.

Further, when a carboxyvinyl polymer is added as a hydrophilic thickener to cosmetics, this polymer has bad compatibility with silica-coated metal oxide fine particles, so that a feel to the skin may be deteriorated in the case where an ultraviolet screening agent for cosmetics is added to a cosmetic and the resulting cosmetic is applied to the skin. However, when the outside of each of the silica-coated metal oxide fine particles is coated with a polymer, compatibility with such a hydrophilic thickener becomes good, so that a feel to the skin is improved in the case where an ultraviolet screening agent for cosmetics is added to a cosmetic and the resulting cosmetic is applied to the skin.

The ultraviolet screening agent for cosmetics according to the present invention contains specific polymer-coated metal oxide fine particles, and therefore, it has excellent dispersibility and excellent storage stability while maintaining high transparency and excellent ultraviolet screening ability and gives cosmetics exhibiting a remarkably improved feel to the skin when added to the cosmetics.

<Polymer-coated metal oxide fine particles>

The polymer-coated metal oxide fine particles are produced by coating, with silica as a first layer, the surface of each of at least one kind of metal oxide fine particles selected from the group consisting of zinc oxide fine particles, titanium oxide fine particles, cerium oxide fine particles, zirconium oxide fine particles, and iron oxide fine particles, and coating the outside of the first layer with a specific polymer as a second layer. Such polymer-coated metal oxide fine particles can be produced in the form of an aqueous dispersion by emulsion polymerization of polymerizable monomers in the presence of silica-coated metal oxide fine particles, preferably silica-coated metal oxide fine particles treated with a coupling agent, in an aqueous medium. Also, when the polymer-coated metal oxide fine particles are separated from the aqueous dispersion and then dried, the polymer-coated metal oxide fine particles can be produced in the form of a powder. Further, when the polymer-coated metal oxide fine particles in the form of a powder are dispersed again in a dispersion medium other than water, or when the dispersion medium of the aqueous dispersion is replaced from water to a dispersion medium other than water, the polymer-coated metal oxide fine particles can be produced in the form of a dispersion in which the polymer-coated metal oxide fine particles are dispersed in a dispersion medium other than water.

As the metal oxide fine particles as described above, commercially available products can be utilized. Also, silica-coated metal oxide fine particles may be prepared per se by any of the heretofore known methods, or commercially available products may be utilized. Although silica-coated metal oxide fine particles can be prepared using, for example, the method described in Japanese Patent Laid-Open (Kokai) Publication No. Hei 11-302015, the most preferred preparation method will be described below.

The silica-coated metal oxide fine particles of the present invention are produced by forming, on the surface of a core material including any one of, or a composite made of two or more of, titanium oxide fine particles, zinc oxide fine particles, iron oxide fine particles, cerium oxide fine particles, and zirconium oxide fine particles, an aqueous silica layer, as a first layer, at from 0.5% by mass to 10% by mass in terms of $SiO_2$, relative to the core material, and, without a drying step, by way of a step of forming an aqueous silica layer, as a second layer, at from 5% by mass to 39.5% by mass in terms of $SiO_2$, relative to the core material, coating the surface of the core material with silica at from 15% by mass to 40% by mass, relative to the core material. This method will be described in detail as shown below in step 1 to step 7.

The following description is made on silica-coated titanium oxide fine particles; however, silica-coated zinc oxide fine particles, silica-coated iron oxide fine particles, silica-coated cerium oxide fine particles, and silica-coated zirconium oxide fine particles can also be produced substantially in the same manner as described for the silica-coated titanium oxide fine particles, with the exception of some parts. Also, chemicals to be used and production conditions described in step 1 to step 7 are mere examples. The present invention is by no means limited thereto, and chemicals indicated in generic concept as described below can be used or conditions indicated by the ranges can be adopted.

Further, preferred conditions and the like described in the production of the silica-coated titanium oxide fine particles can substantially directly be adopted as preferred conditions and the like in the production of silica-coated zinc oxide fine particles, silica-coated iron oxide fine particles, silica-coated cerium oxide fine particles, and silica-coated zirconium oxide fine particles.

Step 1

Strong hydrochloric acid type titania sol of titanium oxide fine particles is prepared. The concentration of the titanium oxide fine particles at this time is 100 g/L; the titanium oxide fine particles in the strong hydrochloric acid type titania sol already have an average primary particle diameter of from 10 to 15 nm, and thus is a high-order dispersion system.

Step 2

An aqueous sodium silicate solution (200 g/L in terms of $SiO_2$) is added to the strong hydrochloric acid type titania sol of the titanium oxide fine particles of step 1 above (at this time, the addition time is not particularly limited, but may be rapid or slow) to form an aqueous silica layer on the surface of each of the titanium oxide fine particles. At this time, the control of the temperature and pH is not particularly needed, and the formation can be carried out at an ordinary temperature and pH. The amount of aqueous silica layer is 3% by mass in terms of $SiO_2$, relative to the titanium oxide particles as the core material (i.e., 3 parts by mass in terms of $SiO_2$, relative to 100 parts by mass of the titanium oxide fine particles). At this time, when the amount of aqueous silica layer formed is greater than 10% by mass, relative to the titanium oxide fine particles as the core material, troubles such as filtration leakage during filtration or a decrease in filtration speed are caused at later steps. The purpose of the formation of the aqueous silica layer in step 2 is to efficiently form an aqueous silica layer onto the particle surface of each of the titanium oxide fine particles with an oligomer of tetramethoxysilane below, so to speak, to have a role of "priming." Also, when the pH changes to be alkaline, the particle surface is strongly negatively charged and the system becomes an extremely strong dispersion system. Thus, as described above, in later steps, filtration leakage during filtration or a decrease in filtration speed is caused.

Step 3

After the formation of the aqueous silica layer in step 2 above, the fine particles are filtered and washed with water. The filtration cake of the aqueous silica-coated titanium oxide fine particles obtained by the filtration has a solid content of from about 40% by mass to 60% by mass. This filtration cake is not dried and it is directly transferred to a next step. The reason why drying is not carried out as in the case is that, when the cake is dried, the aggregation of fine particles proceeds. In the present invention, coating with silica while always avoiding the formation of aggregates of titanium oxide fine particles as much as possible, which is not limited to this step, is the fundamental concept.

Step 4

The above filtration cake and isopropyl alcohol are mixed and stirred using a high-speed dispersing machine (disper). Further, water and aqueous ammonia (having a concentration of 25% by mass) is added thereto, and further stirred. The mixed slurry is strongly dispersed by means of a dyno-mill.

Step 5

A mixed solution of an oligomer of tetramethoxysilane having an average polymerization degree of from 4 to 8 and isopropyl alcohol is gradually added to the above slurry while stirring the slurry using a disper. The addition time is set to 6 hours or longer.

Step 6

After completion of the addition, the slurry is transferred to a kneader tank, and water and isopropyl alcohol are distilled out by heating and pressure reducing. Further, the temperature is raised to 150°C, and curing of the resulting material is carried out. Through these operations, the aqueous silica layer formed on the surface of each of the titanium oxide fine particles is changed into a silica layer.

Step 7

The powder obtained through the above curing is milled by means of a Jet-O-Miser to give silica-coated titanium oxide fine particles in the form of a powder.

The silica-coated metal oxide fine particles of the present invention are produced by way of the steps exemplified in step 1 to step 7 above. An aqueous dispersion obtained by mixing 350 g of the powder obtained and 650 g of deionized water and placing the mixture in a vessel, followed by dispersing using a high-speed dispersing machine at 3,000 rpm for 5 minutes, comes to have a viscosity of 200 mPa.s or lower when viscosity is measured using a B-type viscometer at a rotation rate of 6 rpm at a liquid temperature of 25°C.

In step 1 above, although a case of 100 g/L is exemplified, the present invention is not limited thereto, but may preferably be carried out in the range of from 50 to 300 g/L. Also, in place of strong hydrochloric acid type titania sol of the titanium oxide fine particles used in step 1, a slurry dispersing in water any of zinc oxide fine particles, iron oxide fine particles, cerium oxide fine particles, and zirconium oxide fine particles is used to obtain desired silica-coated metal oxide fine particles. Also, in order to obtain silica-coated zinc oxide fine particles, when an aqueous sodium silicate solution is added in step 2, the temperature and addition speed are left naturally, and the pH is kept in the neutral region (pH from 7 to 8) to need to prevent zinc oxide itself from dissolving. The subsequent steps are allowed to pass through the steps similar to step 3 and the subsequent steps described in the production of the above silica-coated titanium oxide fine particles to obtain silica-coated zinc oxide fine particles.

Thus, in the case of silica-coated zinc oxide fine particles, there are adopted conditions different from those in the production of silica-coated titanium oxide fine particles, such as using zinc oxide fine particles as the starting substance and keeping the pH during the addition of an aqueous alkali silicate solution in the neutral region, but the other conditions can be substantially the same as those in the production of silica-coated titanium oxide fine particles.

In addition, the aqueous silica layer formed as the first layer on the surface of each of metal oxide fine particles as a core material needs to have an amount of from 0.5% by mass to 10% by mass, preferably from 3% by mass to 7% by mass, in terms of $SiO_2$, relative to the metal oxide fine particles. This is because, when the amount of aqueous silica layer is smaller than 0.5% by mass, relative to the metal oxide fine particles, the effect is small as "priming" for efficiently forming the aqueous silica layer onto the particle surface with the oligomer of tetrametoxysilane in the subsequent step, and when the amount of aqueous silica layer is greater than 10% by mass, as described above, in later steps, filtration leakage during filtration or a decrease in filtration speed is caused.

The above hydrated silica layer is formed using an alkali metal silicate in a water system. This is because metal oxide fine particles are relatively readily dispersed in water and an aqueous alkali metal silicate solution is readily available. As the alkali metal silicate, for example, silicate of soda (sodium silicate) or potassium silicate may preferably be used.

The above alkali metal silicate is used in the form of an aqueous solution when added to metal oxide fine particles, and the concentration at that time may preferably be from 100 g/L to 300 g/L in terms of $SiO_2$. The letter "L" as used herein stands for "liter", and the term "liter" is represented by "L" hereinafter as well.

When an aqueous alkali metal silicate solution is added to metal oxide fine particles, the addition time, the temperature of the metal oxide fine particles during the addition, the pH, and the other conditions are not particularly limited. Usually, the addition time may preferably be from 5 minutes to 10 minutes, the temperature may preferably be from 30°C to 60°C, and the pH may preferably be from 0.5 to 4.

Next, on the surface of each of the metal oxide fine particles is formed an aqueous silica layer as a second layer. When the second layer is formed, as exemplified in step 4 above, to the cake obtained in step 3 are added a lower alcohol such as isopropyl alcohol (IPA) and water. The reason why the formation of the second layer is carried out in this manner in the presence of a lower alcohol and water is that a highly hydrophilic lower alcohol such as isopropyl alcohol (IPA) is needed for sufficiently mixing an oligomer of tetraalkoxysilane fed in the next step 5 and water is needed for effectively carrying out the hydrolysis of tetraalkoxysilane. As the lower alcohol, besides isopropyl alcohol exemplified in step 4, there may preferably be used methanol (methyl alcohol), ethanol (ethyl alcohol), butanol (butyl alcohol), benzyl alcohol, and the like. The ratio of the lower alcohol to water (including the water contained in the cake of the titanium oxide fine particles) may preferably be from 3 : 2 to 7 : 2 (mass ratio). The aqueous ammonia added in step 3 is for the purpose of promoting the hydrolysis of tetraalkoxysilane, but is not always necessary.

When the second layer is formed, there is used an oligomer of tetramethoxysilane having an average polymerization degree of 4 to 8 as described in step 5. This is because an oligomer of the tetrametoxysilane is needed for obtaining silica-coated metal oxide fine particles having excellent water dispersibility. In both the cases where the average polymerization degree of an oligomer of the tetrametoxysilane is smaller than 4 and where the average polymerization degree is greater than 8 as well, there cannot be obtained silica-coated metal oxide fine particles having excellent water dispersibility. The average polymerization degree of an oligomer of the tetrametoxysilane is a calculated value of a multiple of the molecular weight of a monomer (in this case, tetramethoxysilane) on the basis of a weight-average molecular weight obtained by gel permeation chromatography (GPC) measurement, assuming that the oligomer is a straight chain.

An oligomer of the tetramethoxysilane is made to be a mixed solution with a lower alcohol such as isopropyl alcohol, when added to the reaction system, as exemplified in step 5. As the lower alcohol, besides isopropyl alcohol exemplified, there may preferably be used methanol, ethanol, butanol, benzyl alcohol, and the like. In a mixed solution with the lower alcohol, an oligomer of the tetramethoxysilane may preferably be set to have a concentration of from about 300 g/L to about 700 g/L.

The formation of an aqueous silica layer with an oligomer of the tetramethoxysilane may preferably be carried out by reacting the oligomer of the tetramethoxysilane under mild conditions. More specifically, a mixed solution of an oligomer of the tetramethoxysilane and a lower alcohol may preferably be reacted while they are added to the reaction system. From the viewpoint, the addition time of a mixed solution of an oligomer of the tetramethoxysilane and a lower alcohol to the reaction system is not particularly limited, but may usually preferably be from about 4 hours to about 8 hours. On the basis of the addition of a mixed solution of an oligomer of the tetramethoxysilane and a lower alcohol, an aqueous silica layer as a second layer is formed, and an aqueous silica layer as a second layer and an aqueous silica layer formed as a first layer are changed into silica layers by subsequent heating, pressure reducing, and curing. An aqueous silica layer formed as the second layer is needed to be from 5% by mass to 39.5% by mass in terms of $SiO_2$, relative to the metal oxide fine particles as the core material (i.e., from 5 to 39.5 parts by mass in terms of $SiO_2$, relative to 100 parts by mass of the metal oxide fine particles). This is because, when the second aqueous silica layer is smaller than 5% by mass, relative to the metal oxide fine particles as the core material, the amount of silica coating the surface of each of the metal oxide fine particles is decreased and various characteristics including water dispersability are deteriorated, thereby lowering the polymerization stability during polymer coating. Also, this is because, when the second aqueous silica layer is at an amount of greater than 39.5% by mass, no effect accompanying such an increase in the amount of aqueous silica is observed, the excess is not economical, and also filtering properties are lowered.

The curing as described in step 6 is carried out for the purpose of completely removing water and a lower alcohol such as isopropyl alcohol, both of which cannot be distilled out only by heating and pressure reducing. The temperature at that time is not limited to 150°C exemplified. Also, the powder obtained is usually pulverized to make the silica-coated titanium oxide fine particles in the form of a power.

Any of the silica-coated metal oxide fine particles obtained in this manner has a water dispersability of 200 mPa.s or smaller in the above specific viscosity notation, as in the case of the silica-coated titanium oxide fine particles, and can preferably be used in the subsequent polymer coating step.

The shape of the metal oxide fine particles is not particularly limited, but it may be, for example, granules such as spherical, ellipsoidal, and polygonal; flakes such as scale-like and (hexagonal) plate like; a needle shape, a columnar shape, a rod shape, a tubular shape, and the like. These shapes may exist alone, or two or more kinds of these shapes may also exist in combination. In these shapes, preferred are granules such as spherical, ellipsoidal, and polygonal.

The number-average particle diameter of the metal oxide fine particles is usually not smaller than 1 nm and not greater than 100 nm, preferably not smaller than 5 nm and not greater than 80 nm, more preferably not smaller than 8 nm and not greater than 60 nm, and still more preferably not smaller than 10 nm and not greater than 50 nm. When the number-average particle diameter of the metal oxide fine particles is smaller than 1 nm, the metal oxide fine particles are aggregated to form a high-order structure in some cases, so that it becomes difficult to obtain polymer-coated metal oxide fine particles having a specified number-average particle diameter. In contrast, the number-average particle diameter of the metal oxide particles is greater than 100 nm, the number-average particle diameter of polymer-coated metal oxide fine particles becomes great in some cases, so that a transparent feel when a cosmetic containing the polymer-coated metal oxide fine particles is applied to the skin may be decreased.

In the present invention, the number-average particle diameter of the metal oxide fine particles is a value measured by the method described below in Examples. The term "primary particle diameter" as used herein means the particle diameter of the shortest portion of a primary particle, unless otherwise noted, and the term "the particle diameter of the shortest portion" as used herein means the shortest length passing the center of a primary particle. For example, when the shape of the metal oxide fine particles is spherical, the particle diameter of the shortest portion means the diameter of the sphere, and when the shape of the metal oxide fine particles is ellipsoidal, the particle diameter of the shortest portion means the short diameter in the short diameter and long diameter. When the shape of the metal oxide fine particles is polygonal, the particle diameter of the shortest portion means the shortest length passing through the center of a primary particle, and when the shape of the metal oxide fine particles is flaky such as scale-like and (hexagonal) planar, the particle diameter of the shortest portion means the shortest length (i.e., thickness) passing through the center of a primary particle in the direction (i.e., the thickness direction) perpendicular to the in-plane direction. When the shape of the metal oxide fine particles is a needle shape, a columnar shape, a rod shape, a tubular shape, or the like, the particle diameter of the shortest portion means the shortest length passing through the center of a primary particle, which shortest length is measured in a direction perpendicular to the length direction.

In the polymer-coated metal oxide fine particles of the present invention, the surface of each of the metal oxide fine particles is coated with a specific polymer. The phrase "coated with a polymer" as used herein means that the whole surface of each of the metal oxide fine particles is coated with a polymer in seamless manners. The polymer coating the surface of each of the metal oxide fine particles is referred to sometimes as the "coasting polymer". The coating polymer covers the surface of each of the metal oxide fine particles by the emulsion polymerization of a polymerizable monomer in the presence of the metal oxide fine particles, preferably the metal oxide fine particles treated with a coupling agent in an aqueous medium. The coating polymer includes (meth)acrylic-type polymers, styrene-type polymers, vinyl acetate-type polymers, vinyl chloride-type polymers, vinylidene-type polymers, and their copolymers. These polymers may be used alone, or two or more kinds of these polymers may also be used in combination. In these polymers, preferred are (meth)acrylic-type polymers, styrene-type polymers, and their copolymers.

The polymer-coated metal oxide fine particles may be coated with a single polymer or may be coated with two or more kinds of polymers. Also, the polymer-coated metal oxide fine particles may be composed of one kind of fine particles with the same coating polymer or may be composed of two or more kinds of fine particles with different coating polymers.

When the metal oxide fine particles treated with a coupling agent in advance of emulsion polymerization are used, the coating polymer is chemically bonded, through the coupling agent, to the surface of each of the metal oxide fine particles in the polymer-coated metal oxide fine particles obtained. The term "chemical bond" as used herein mainly means a covalent bond, but for example, since a covalent bond between different atoms has occasionally the characteristic of an ionic bond in a greater or less degree, the "chemical bond" as used in the present invention may further include a case where the covalent bond and the ionic bond are in resonance with each other. However, the "chemical bond" as used in the present invention does not include weak bonds which act between molecules, such as static attractive forces, dispersion forces, hydrogen bonds, and charge-transfer forces. Also, the phrase "chemically bonded, through a coupling agent, to ..." as used herein means that a hydroxyl group existing on the surface of each of the metal oxide fine particles is chemically bonded to the coupling agent and the coupling agent is chemically bonded to the coating polymer.

When the metal oxide fine particles treated with a coupling agent in advance of emulsion polymerization are used, the polymer-coated metal oxide fine particles exhibits excellent water resistance because the coating polymer is chemically bonded, through the coupling agent, to the surface of each of the metal oxide fine particles, so that the coating polymer is firmly attached to each of the metal oxide fine particles, thereby not allowing water and the like to enter between each of the metal oxide fine particles and the coating polymer. Thus, for example, when silica-coated zinc oxide fine particles are used, the elution of zinc oxide hardly occurs.

The silica-coated metal oxide fine particles used in the production of polymer-coated metal oxide fine particles may be prepared per se by any of the heretofore known methods or commercially available products may be utilized, as described above.

<Production of polymer-coated metal oxide fine particles>

The aqueous dispersion of polymer-coated metal oxide fine particles can be produced by carrying out the emulsion polymerization using a polymerizable monomer and a radical initiator in the presence of silica-coated metal oxide fine particles.

The silica-coated metal oxide fine particles may preferably be treated with a coupling agent in advance of the emulsion polymerization. The treatment of the silica-coated metal oxide fine particles with a coupling agent makes it possible to react a hydroxyl group existing on the surface of each of the silica-coated metal oxide fine particles with the coupling agent, so that a functional group can be introduced through a chemical bond on the surface of each of the silica-coated metal oxide fine particles. After a functional group is introduced on the surface of each of the silica-coated metal oxide fine particles, a polymerizable monomer having a reactive group which can be reacted with the functional group is reacted with the metal oxide fine particles, and a polymer is synthesized from the polymerizable monomer on the surface of each of the silica-coated metal oxide fine particles, so that the surface of each of the silica-coated metal oxide fine particles can be coated with the polymer in seamless manners.

The coupling agent is not particularly limited, so long as it is a compound having a reactive site reacting with a hydroxyl group existing on the surface of each of the metal oxide fine particles and a functional group reacting with a reactive group of the polymerizable monomer having the reactive group, but examples thereof may include silane coupling agents and titanate type coupling agents having various functional groups. When a silane coupling agent is used, various functional groups are introduced through an -O-Si- bond on the surface of each of the silica-coated metal oxide fine particles by reacting with a hydroxyl group existing on the surface of each of the silica-coated metal oxide fine particles. Also, when a titanate type coupling agent is used, various functional groups are introduced through an -O-Ti- bond on the surface of each of the silica-coated metal oxide fine particles. As the coupling agent, silane coupling agents are preferred because silane coupling agents having various functional groups are commercially available and therefore can easily be obtained. Examples of the functional group contained in the coupling agent may include a vinyl group, a (meth)acryloyl group, an epoxy group, an amino group, an isocyanate group, and a mercapto group.

The silane coupling agent is not particularly limited, so long as it is a silane coupling agent containing, for example, a vinyl group, a (meth)acryloyl group, an epoxy group, an amino group, an isocyanate group, or a mercapto group, but examples thereof may include vinyl group-containing silane coupling agents such as vinyltrimethoxysilane, vinyldimethylmethoxysilane, vinyltrichlorosilane, and vinyldimethylchlorosilane; (meth)acryloyl group-containing silane coupling agents such as γ-(meth)acryloxypropyltrimethoxysilane, γ-(meth)acryloxypropyltriethoxysilane, γ-(meth)acryloxypropylmethyldimethoxysilane, γ-(meth)acryloxypropylmethyldiethoxysilane, and N-β-(N-vinylbenzylaminoethyl)-γ-aminopropyltrimethoxysilane; epoxy group-containing silane coupling agents such as β-(3,4-epoxycyclohexyl)-ethyltrimethoxysilane, β-(3,4-epoxycyclohexyl)-ethyltriethoxysilane, β-(3,4-epoxycyclohexyl)-ethyltriisopropoxysilane, β-(3,4-epoxycyclohexyl)-ethylmethyldimethoxysilane, β-(3,4-epoxycyclohexyl)ethylmethyldiethoxysilane, γ-glycidoxypropyltrimethoxysilane, γ-glycidoxypropyltriethoxysilane, γ-glycidoxypropyltriisopropoxysilane, γ-glycidoxypropylmethyldimethoxysilane, and γ-glycidoxypropylmethyldiethoxysilane; amino group-containing silane coupling agents such as γ-aminopropyltrimethoxysilane, γ-aminopropyltriethoxysilane, γ-aminopropylmethyldimethoxysilane, γ-aminopropylmethyldiethoxysilane, N-(β-aminoethyl)-γ-aminopropyltrimethoxysilane, N-(β-aminoethyl)-γ-aminopropyltriethoxysilane, N-(β-aminoethyl)-γ-aminopropylmethyldimethoxysilane, N-(β-aminoethyl)-γ-aminopropylmethyldiethoxysilane, N-phenyl-γ-aminopropyltrimethoxysilane, and N-phenyl-γ-aminopropyltriethoxysilane; isocyanate group-containing silane coupling agents such as γ-isocyanopropyltrimethoxysilane, γ-isocyanopropyltriethoxysilane; γ-isocyanopropylmethyldimethoxysilane, and γ-isocyanopropylmethyldiethoxysilane; and mercapto group-containing silane coupling agents such as γ-mercaptopropyltrimethoxysilane. These silane coupling agents may be used alone, or two or more kinds of these silane coupling agents may also be used in combination. In these silane coupling agents, vinyl group-containing silane coupling agents and (meth)acryloyl group-containing silane coupling agents are preferred because polymer synthesis can efficiently be carried our from the surface of each of the silica-coated metal oxide fine particles.

To treat the silica-coated metal oxide fine particles with the coupling agent, for example, the silica-coated metal oxide fine particles and the coupling agent may be mixed and stirred in an aqueous medium. At that time, to promote the reaction of the silica-coated metal oxide fine particles with the coupling agent, they can preferably be warmed or heated, if necessary, to a temperature of not lower than 30°C and not higher than 100°C, more preferably not lower than 40°C and not higher than 80°C. The amount of coupling agent to be used may preferably be not smaller than 0.05% by mass and not greater than 20% by mass, more preferably not smaller than 0.1% by mass and not greater than 15% by mass or less, and still more preferably not smaller than 0.5% by mass and not higher than 10% by mass or less, relative to the silica-coated metal oxide fine particles. When the amount of coupling agent to be used is smaller than 0.05% by mass, the surface of each of the silica-coated metal oxide fine particles cannot sufficiently be coated with a polymer. In contrast, when the amount of coupling agent to be used is greater than 20% by mass, the viscosity of the reaction solution may be increased, and the reaction solution may cause gelation.

The aqueous medium to be used when the silica-coated metal oxide fine particles are treated with the coupling agent is similar to the aqueous medium to be used for polymerization reaction explained below, and it may be the same as, or different from, the aqueous medium to be used for polymerization reaction.

When the silica-coated metal oxide fine particles are treated with the coupling agent, the silica-coated metal oxide fine particles may preferably be dispersed in the aqueous medium; therefore, a dispersion stabilizer can be used, if necessary. Examples of the dispersion stabilizer may include heretofore known surfactants and polymer dispersion stabilizers such as POVAL. These dispersion stabilizers may be used alone, or two or more kinds of these dispersion stabilizers may also be used in combination. The amount of dispersion stabilizer to be used may preferably be at least 0% by mass and not greater than 5% by mass, more preferably at least 0% by mass and not greater than 4% by mass, and still more preferably at least 0% by mass and not greater than 3% by mass or less, relative to the aqueous medium. When the amount of dispersion stabilizer to be used is greater than 5% by mass, the silica-coated metal oxide fine particles cannot efficiently be treated with the coupling agent.

In the case of a coupling agent having a polymerizable reactive group, when an unreacted coupling agent exists after the silica-coated metal oxide fine particles are treated with the coupling agent, polymerization stability in the polymerization step is lowered, so the silica-coated metal oxide particles aggregate in some cases. Therefore, after the silica-coated metal oxide fine particles are treated with the coupling agent, the silica-coated metal oxide fine particles treated with the coupling agent can be washed for removing the unreacted coupling agent. For washing the silica-coated metal oxide fine particles treated with the coupling agent, for example, they may be dispersed again in an appropriate solvent and subjected to centrifugal separation, and supernatant liquid is discarded and only the precipitate is collected. The operation including re-dispersion, centrifugal separation, and collection of only the precipitate is not always carried out from an economical point of view. When this operation is carried out, it may be repeated only once or more than once, but may preferably be repeated three times or more.

The polymerization reaction is carried out in an aqueous medium in the presence of the silica-coated metal oxide fine particles, preferably the silica-coated metal oxide fine particles treated with the coupling agent. When the polymerization reaction is carried out in the presence of the silica-coated metal oxide fine particles treated with the coupling agent, the dispersion obtained by treating the silica-coated metal oxide fine particles with the coupling agent may be used, as it is, for the polymerization reaction, or the dispersion obtained by dispersing the silica-coated metal oxide fine particles again in an aqueous medium after treatment with the coupling agent may be used.

The polymerizable monomer to be used for the polymerization reaction may appropriately be selected from the polymerizable monomers having a reactive group which can be reacted with a functional group introduced on the surface of each of the silica-coated metal oxide fine particles, depending on the functional group, and it is not particularly limited, but examples thereof may include polymerizable monomers having a reactive group which can be reacted with a functional group such as a vinyl group, a (meth)acryloyl group, an epoxy group, an amino group, an isocyanate group, or a mercapto group, for example, polymerizable monomers having a vinyl group, a (meth)acryloyl group, an epoxy group, an amino group, a carboxyl group, a hydroxyl group, or the like. These polymerizable monomers may be used alone, or two or more kinds of these polymerizable monomers may also be used in combination.

Examples of the polymerizable monomer having a vinyl group may include halogenated vinyl compounds such as vinyl chloride and vinylidene chloride; vinyl esters such as vinyl acetate; and styrene derivatives such as styrene, α-methylstyrene, vinyl toluene, and chlorostyrene. These polymerizable monomers may be used alone, or two or more kinds of these polymerizable monomers may also be used in combination. In these polymerizable monomers, styrene derivatives such as styrene are preferred.

Examples of the polymerizable monomer having a (meth)acryloyl group may include (meth)acrylates such as methyl (meth)acrylate, ethyl (meth)-acrylate, butyl (meth)acrylate, 2-ethylhexyl (meth)acrylate, and cyclohexyl (meth) acrylate. These polymerizable monomers may be used alone, or two or more kinds of these polymerizable monomers may also be used in combination. In these polymerizable monomers, (meth)acrylates such as methyl (meth)acrylate, butyl (meth)acrylate, and cyclohexyl (meth)acrylate are preferred.

Examples of the polymerizable monomer having an amino group may include (meth)acrylates such as aminoethyl (meth)acrylate, dimethylaminoethyl (meth)acrylate, and dimethylaminopropyl (meth)-acrylate; vinyl amines such as N-vinyldiethylamine and N-acetylvinylamine; allylamine compounds such as α-methylallylamine and N,N-dimethylallylamine; (meth)acrylamide compounds such as (meth)acrylamide, N-methyl(meth)acrylamide, and N,N-dimethyl-(meth) acrylamide; and aminostyrene compounds such as p-aminostyrene. These polymerizable monomers may be used alone, and two or more kinds of these polymerizable monomers may also be used in combination. In these polymerizable monomers, (meth)acrylates such as aminoethyl (meth)acrylate and dimethylaminoethyl (meth)acrylate are preferred.

Examples of the polymerizable monomer having an epoxy group may include unsaturated carboxylic acid esters such as glycidyl (meth)acrylate; and unsaturated glycidyl ethers such as vinyl glycidyl ether and allyl glycidyl ether. These polymerizable monomers may be used alone, or two or more kinds of these polymerizable monomers may also be used in combination. In these polymerizable monomers, unsaturated carboxylic acid esters such as glycidyl (meth) acrylate are preferred.

Examples of the polymerizable monomer having a carboxyl group may include unsaturated monocarboxylic acids such as (meth)acrylic acid and crotonic acid; unsaturated dicarboxylic acids such as maleic acid, itaconic acid, and citraconic acid; monoester compounds of these unsaturated dicarboxylic acids; and anhydrides of these unsaturated dicarboxylic acids. These polymerizable monomers may be used alone, or two or more kinds of these polymerizable monomers may also be used in combination. In these polymerizable monomers, unsaturated monocarboxylic acids such as (meth)acrylic acid are preferred.

Examples of the polymerizable monomer having a hydroxyl group may include (meth)acrylates such as 2-hydroxyethyl (meth)acrylate, 2-hydroxypropyl (meth)acrylate, methyl α-hydroxymethylacrylate, and ethyl α-hydroxymethacrylate; polycaprolactone-modified (meth)acrylates; and polyoxyethylene-modified and polyoxypropylene-modified (meth)acrylates. These polymerizable monomers may be used alone, or two or more kinds of these polymerizable monomers may also be used in combination. In these polymerizable monomers, (meth)acrylates such as 2-hydroxyethyl (meth)-acrylate and 2-hydroxypropyl (meth)acrylate are preferred.

The amount of polymerizable monomer to be used may appropriately be selected depending on the amount of silica-coated metal oxide fine particles to be used, and it is not particularly limited, but may preferably be not smaller than 1 part by mass and not greater than 200 parts by mass, more preferably not smaller than 2 parts by mass and not greater than 100 parts by mass, and still more preferably not smaller than 5 parts by mass and not greater than 50 parts by mass, relative to 100 parts by mass of the silica-coated metal oxide fine particles. When the amount of polymerizable monomer to be used is smaller than 1 part by mass, polymerization reaction cannot proceed smoothly and the surface of each of the silica-coated metal oxide fine particles cannot efficiently be coated with a polymer. In contrast, when the amount of polymerizable monomer to be used is greater than 200 parts by mass, many polymer particles not containing the silica-coated metal oxide fine particles may be prepared.

The polymerization initiator is not particularly limited, so long as it is a watersoluble radical polymerization initiator, but may include peroxides such as hydrogen peroxide, potassium persulfate, potassium persulfate, sodium persulfate, ammonium persulfate, and potassium perphosphorate; redox initiators in which these peroxides are combined with reducing agents such as ascorbic acid and its salt, erythorbic acid and its salt, tartaric acid and its salt, citric acid and its salt, sodium thiosulfate, sodium hydrogensulfite, sodium pyrrosulfite, Rongalit C (NaHSO$_2$. CH$_2$O. H$_2$O), Rongalit Z (ZnSO$_2$.CH$_2$O.H$_2$O), and Dechroline (Zn(HSO$_2$.CH$_2$O)$_2$); hydroperoxides such as t-butyl hydroperoxide, t-amyl hydroperoxide, t-hexyl hydroperoxide, p-menthane hydroperoxide, and cumene hydroperoxide; dialkyl peroxides such as di-t-butyl peroxide and di-t-amyl peroxide; diacyl peroxide such as dibenzoyl peroxide, dioctanoyl peroxide, didecanoyl peroxide, and didodecanoyl peroxide; peroxy esters such as t-butylperoxy pivalate, t-amylperoxy pivalate, and t-butylperoxy benzoate; and azo compounds such as 2,2'-azobis-(isobutyronitrile), 2,2'-azobis(2-methylbutyronitrile), 2,2'-azobis (2,4-dimethylvaleronitrile), 2,2'-azobis(4-methoxy-2,4-dimethylvaleronitrile), dimethyl 2,2'-azobis(isobutyrate), 4,4'-azobis(4-cyanovaleric acid), 2,2',-azobis(2-methylpropiondiamine) dihydrochloride, 2,2'-azobis[N-(2-carboxyethyl)-2-methylpropiondiamine] n-hydrate, 2,2'-azobis{2-methyl-N-[2-(1-hydroxybutyl)]-propionamide}, 2,2'-azobis{2-methyl-N-[1,1-bis(hydroxymethyl-2-hydroxyethyl)propionamide], 2,2'-azobis(1-imino-1-pyrrolidino-2-ethylpropane) dihydrochloride, 2,2'-azobis[2-(2-imidazolin-2-yl)propane], 2,2'-azobis[2-(2-imidazolin-2-yl)-propane] dihydrochloride, 2,2'-azobis[2-(2-imidazolin-2-yl)propane] disulfate dihydrate, 2,2'-azobis{2-[1-(2-hydroxyethyl)-2-imidazolin-2-yl]propane} dihydrochloride, 2,2'-azobis[2-(5-methyl-2-imidazolin-2-yl)propane] dihydrochloride, and 1,1'-azobis(cyclohexane-1-carbonitrile). These polymerization initiators may be used alone, or two or more kinds of these polymerization initiators may also be used in combination.

The amount of polymerization initiator to be used may appropriately be adjusted depending on the amount of polymerizable monomer to be used, and it is not particularly limited, but may preferably be not smaller than 0.001% by mass and not greater than 3% by mass, more preferably not smaller than 0.005% by mass and not greater than 2% by mass, and still more preferably not smaller than 0.01% by mass and not greater than 1% by mass, relative to the polymerizable monomer.

The polymerization reaction of the monomer components is carried out in the aqueous medium. The term "aqueous medium" as used herein means water or a mixed solvent of water and a water-miscible organic solvent. When a mixed solvent of water and a water-miscible organic solvent is used as the aqueous medium, the monodisperse state of the silica-coated metal oxide fine particles as a raw material and the polymer-coated metal oxide fine particles prepared can be kept sufficiently well without using a dispersion stabilizer such as a surfactant. However, when it is not desirable that an organic solvent is contaminated in the aqueous dispersion of polymer-coated metal oxide fine particles or in the ultraviolet screening agent, the monodisperse state of the silica-coated metal oxide fine particles as a raw material and the polymer-coated metal oxide fine particles prepared can be kept sufficiently well by using a dispersion stabilizer.

When a mixed solvent of water and a water-miscible organic solvent is used as the aqueous medium, a ratio of a water-miscible organic solvent to water may preferably be at least 0% by mass and not greater than 40% by mass, more preferably at least 0% by mass and not greater than 20% by mass.

Examples of the water-miscible organic solvent which can be used in combination with water may include alcohols such as methanol, ethanol, isopropyl alcohol, n-propyl alcohol, and allyl alcohol; glycols such as ethylene glycol, propylene glycol, butylene glycol, hexylene glycol, pentanediol, hexanediol, heptanediol, and dipropylene glycol; ketones such as acetone, methyl ethyl ketone, and methyl propyl ketone; esters such as methyl formate, ethyl formate, methyl acetate, and methyl acetoacetate; and ethers such as diethylene glycol monomethyl ether, diethylene glycol monoethyl ether, diethylene glycol dimethyl ether, ethylene glycol monomethyl ether, ethylene glycol monoethyl ether, and dipropylene glycol monomethyl ether. These organic solvents may be used alone, or two or more kinds of these organic solvents may also be used in combination. In these organic solvents, preferred are organic solvents which become poor solvents for a polymer synthesized from monomer components, that is, which dissolve the monomer components but do not dissolve a polymer synthesized from the monomer components.

The reaction temperature at which the polymerization reaction is carried out is not particularly limited, but may preferably be not lower than 40°C and not higher than 90°C, more preferably not lower than 50°C and not higher than 80°C. Also, the reaction time may appropriately be adjusted depending on the amounts of silica-coated metal oxide fine particles and polymerizable monomer to be used, and it is not particularly limited, but may preferably be not shorter than 1 hour and not longer than 24 hours, more preferably not shorter than 3 hours and not longer than 12 hours.

After the polymerization reaction, an aqueous dispersion of polymer-coated metal oxide fine particles is obtained, in which the surface of each of the silica-coated metal oxide fine particles is coated with a specific polymer. The aqueous dispersion obtained may be used, as it is, or for example, the polymerization reaction solution is subjected to centrifugal separation to separate supernatant liquid and precipitate, which precipitate is collected and dried to give the polymer-coated metal oxide fine particles, and they may be used as a powder. The method of drying the polymer-coated metal oxide fine particles may appropriately be selected from the heretofore known drying methods, and it is not particularly limited, but examples thereof may include natural drying, heating drying, reduced-pressure drying, and spray drying. The polymer-coated metal oxide fine particles obtained may be used as a powder itself or may be used as a dispersion in which the polymer-coated metal oxide fine particles are dispersed again in an appropriate solvent.

The method of dispersing the polymer-coated metal oxide fine particles again in a dispersion medium may appropriately be selected from the heretofore known drying methods, and it is not particularly limited, but examples thereof may include methods using a stirrer, a ball mill, a sand mill, or an ultrasonic homogenizer.

Also, when the polymer-coated metal oxide fine particles are in the form of a dispersion and the polymer-coated metal oxide fine particles are dispersed in a different dispersion medium, there can be used a method in which the polymer-coated metal oxide fine particles are separated, for example, by filtration, centrifugal separation, or evaporation of the dispersion medium, and then mixed with a dispersion medium to be replaced, followed by dispersing the mixture using any of the methods described above, or what is called a solvent replacement method with heating, in which the dispersion is heated so that part or all of the dispersion medium constituting the dispersion is evaporated and distilled out, while a dispersion medium to be replaced is mixed therein.

«Cosmetics»

The cosmetic of the present invention is obtained by adding an ultraviolet screening agent for cosmetics obtained as described above. The ultraviolet screening agent for cosmetics is added in a form appropriately selected depending on the form of a cosmetic containing the ultraviolet screening agent. For example, when the cosmetic is in a powder form like a powder foundation, the ultraviolet screening agent is added in a powder form; when the cosmetic is in an oil-based agent form like a lip stick or an oil-based foundation, the ultraviolet screening agent is added in the form of a powder and/or a dispersion using a dispersion medium other than water; and when the cosmetic is in an emulsion form like an emulsified foundation, a cream, or a gel, the ultraviolet screening agent is added in the form of a powder, and/or an aqueous dispersion using water as a dispersion medium, and/or a dispersion using a dispersing agent other than water.

The amount of ultraviolet screening agent for cosmetics to be added is normally from 1% by mass to 80% by mass, relative to the total mass of the cosmetic, and can appropriately be selected depending on the form of the cosmetic. In other words, preferably, the amount of ultraviolet screening agent is normally from 40% by mass to 80% by mass in a powder form like a powder foundation, normally from 1% by mass to 20% by mass in an oil-based agent form like a lip stick, or an oil-based foundation, normally from 1% by mass to 40% by mass in an emulsification form like an emulsion foundation, a cream, or a gel. These cosmetics are produced by any of the ordinary methods and are provided for each of the intended purposes.

Further, regardless of the form of an ultraviolet screening agent for cosmetics, the amount of polymer-coated metal oxide particles to be added, as a solid content in a cosmetic, may preferably be not smaller than 1% by mass and not greater than 40% by mass, more preferably not smaller than 1.5% by mass and not greater than 35% by mass, and still more preferably not smaller than 2% by mass and not greater than 30% by mass, relative to the total mass of the cosmetic. When the amount of polymer-coated metal oxide particles to be added is smaller than 1% by mass, the ultraviolet screening ability of a cosmetic containing the ultraviolet screening agent for cosmetics becomes insufficient, thereby making impossible the effective screening of ultraviolet rays in some cases. In contrast, when the amount of polymer-coated metal oxide particles to be added is greater than 40% by mass, the polymer-coated metal oxide fine particles are aggregated to form a high-order structure, so that the dispersibility and storage stability of a cosmetic containing the ultraviolet screening agent for cosmetics are decreased in some cases.

In the cosmetic of the present invention, for example, an cosmetic in the form of an emulsion, such as an emulsion foundation, a cream, and a gel, a carboxyvinyl polymer may preferably be added as a hydrophilic thickener or an emulsion stabilizer. In the case where a carboxyvinyl polymer is added to a cosmetic, the amount of carboxyvinyl polymer to be added may preferably be not smaller than 0.01% by mass and 5% by mass, more preferably not smaller than 0.1% by mass and 3% by mass, relative to the total mass of the cosmetic. When the amount of carboxyvinyl polymer to be added is smaller than 0.01% by mass, the cosmetic does not become a sufficient gel state in some cases. In contrast, when the amount of carboxyvinyl polymer to be added is greater than 5% by mass, the viscosity of the cosmetic may increase more than necessary.

Also, the cosmetic of the present invention may appropriately contain, in a range without damaging the effect of the present invention, heretofore known components such as surfactants such as sorbitan fatty acid esters, polyglycerin fatty acid esters, sucrose fatty acid esters, and polyoxyethylenealkyl ethers; hydrocarbons such as squalane, liquid paraffin, and paraffin wax; fatty acid esters such as isopropyl palmitate and butyl stearate; silicone oils such as dimethicone and cyclomethicone; oil agents such as bees wax, olive oil, and sunflower oil; polyhydric alcohols such as glycerin, propylene glycol, and butylene glycol; stabilizers such as sodium chloride and magnesium sulfate; vitamins such as vitamin A, vitamin B families, vitamin C, and vitamin E; anti-inflammatory agents such as amino acids, glycyrrhizinate salts, and glycyrrhetinate salts; organic ultraviolet absorbers such as those of the PABA type, e.g., paradimethylaminobenzoic acid, those of the cinnamic acid type, e.g., octyl methoxycinnamate, those of the benzophenone type, e.g., oxybenzones, and those of the salicylic acid type; perfumes; dyes; pigments; antiseptic agents; antioxidants; astringent drugs; cell activators; skin-whitening agents; moisturizing agents; skin roughness improvers; and beauty components, thereby making it possible to prepare cosmetics, particularly cosmetics for the purpose of preventing sunburn, such as foundations, lip sticks, lip creams, oil foundations, milky lotions, creams, and gels.

The cosmetic of the present invention contains an ultraviolet screening agent for cosmetics as mentioned above, and therefore, it has high transparency and excellent ultraviolet screening ability and exhibits an improved feel to the skin.

EXAMPLES

The present invention will be explained below in detail by reference to Examples, but the present invention is not limited to these Examples. The present invention can be put into practice after appropriate modifications or variations within a range meeting the gists described above and later, all of which are included in the technical scope of the present invention.

With respect to the dispersions or powders of polymer-coated metal oxide fine particles obtained in the following Production Examples, the shapes and number-average particle diameters of fine particles contained were determined or measured by the methods described below. When powderization was required before determination and measurement, powderization was carried out according to the method described below and then, if not otherwise specified, a powder obtained was used as a measurement sample.

<Shape>

The shape of polymer-coated metal oxide fine particles was determined by observing the fine particles with a scanning electron microscope or a transmission electron microscope (magnification: 10,000-fold).

<Primary particle diameter and number-average particle diameter>

Primary particle diameters of arbitrary one hundred fine particles contained in a photographed image which was obtained by observing metal oxide fine particles or polymer-coated metal oxide fine particles with a scanning electron microscope or a transmission electron microscope (magnification: 10,000-fold) were measured, and the number-average particle diameter was calculated by the following numerical formula. Further, when the fine particles were observed with the scanning electron microscope, the deposition treatment with a noble metal alloy was carried out to the fine particles in advance for observation; therefore, the number-average particle diameter was determined with a correction on the thickness of a deposition layer.

$$d_n = \left( \sum_{i=1}^{n} D_i \, / \, n \right)$$

wherein $d_n$ represents the number-average particle diameter, Di represents the primary particle diameter of the i-th fine particle, and n represents the number of the fine particles.

First, the following will describe Production Examples of silica-coated metal oxide fine particles produced by coating the surface of each of metal oxide fine particles with silica, particularly silica-coated titanium oxide fine particles and silica-coated zinc oxide fine particles.

«Production Example 1»

Aqueous titanium oxide obtained by the heretofore known method, i.e., hydrolysis of titania sulfate, was treated with caustic alkali and cured under heating in hydrochloric acid to give a strong hydrochloric acid type titania sol (having a $TiO_2$ concentration of 140 g/L) containing titanium oxide fine particles with a rutile type crystal structure, having an average particle diameter of 150 nm, which titania sol was adjusted to a concentration of 100 g/L. Then, 10 L (1,000 g in terms of $TiO_2$) of the strong hydrochloric acid type titania sol was taken by measurement. To this strong hydrochloric acid type titania sol was added 150 mL of an aqueous sodium silicate solution (already adjusted to a concentration of 200 g/L in terms of $SiO_2$) (3% by mass in terms of $SiO_2$, relative to the titanium oxide fine particles as a core material) while the strong hydrochloric acid type titania sol was stirred at room temperature, and the mixture was cured for 15 minutes. The slurry after the curing was filtered and washed with water. Thus, a filtered cake was obtained, which contained 50% by mass of the titanium oxide fine particles, on the surface of each of the titanium oxide fine particles as the core material being formed, as a first layer, an aqueous silica layer at 3% by mass in terms of $SiO_2$, relative to the core material. This filtered cake was subjected to a next step without drying.

To 2,060 g of the resulting filtered cake was added 5,000 g of isopropyl alcohol, followed by mixing with stirring using a disper to form a slurry. Further, to this slurry were added 1,000 g of deionized water and 50 g of aqueous ammonia, followed by further mixing with stirring (at this time, the pH of the mixed solution was set to be from 10 to 11; and the amount of ammonia was regulated for adjustment of pH).

The resulting slurry was transferred to a horizontal bead mill (DYNO-MILL ECO-5, available from Willy A. Bachofen AG) at 150 mL/min. To this slurry were gradually added over 6 hours a mixed solution of 423 g of an oligomer of tetramethoxysilane having an average polymerization degree of about 5 (MKC Silicate MS51, available from Mitsubishi Chemical Corp.; having a molecular weight of 500 to 700 and a $SiO_2$ content of 52% by mass) and 212 g of isopropyl alcohol (the concentration of tetramethoxysilane in the mixed solution was 346 g/L) while the slurry was stirred within the horizontal bead mill to form, as a second layer, an aqueous silica layer at 22% by mass in terms of $SiO_2$, relative to the titanium oxide fine particles as the core material, on the aqueous silica layer as the above first layer. The addition speed at this time was 1.76 g/min. In the slurry after the addition of such an oligomer of tetramethoxysilane, neither remarkable thickening nor whitening was observed.

Then, the resulting slurry was transferred to a heating vacuum kneader, and water and isopropyl alcohol were distilled out by heating and pressure reducing. Thereafter, the residue was further heated to 150°C and then kept at the temperature for 2 hours to carry out curing. The powder obtained by such curing was pulverized with a JO mill (SK JET-O-MILL, available from Seishin Enterprise Co., Ltd.) to give silica-coated titanium oxide fine particles. The amount of silica coating of the silica-coated titanium oxide fine particles was 25% by mass in terms of $SiO_2$, relative to the titanium oxide fine particles as the core material. Then, 350 g of the resulting silica-coated titanium oxide fine particles and 650 g of deionized water were placed in a vessel, and the mixture was dispersed using a high-speed dispersing machine at 3,000 rpm for 5 minutes to give an aqueous dispersion of the silica-coated titanium oxide fine particles. This aqueous dispersion of the silica-coated titanium oxide fine particles was measured by a B-type viscometer (at 6 rpm at a solution temperature of 25°C), and it was found to have a viscosity of 10 mPa.s. Finally, 750 g of deionized water was added thereto to give an aqueous dispersion (TS-1) of the silica-coated titanium oxide fine particles. This aqueous dispersion (TS-1) of the silica-coated titanium oxide fine particles had a 20% non-volatile content. The titanium oxide fine particles had a primary particle diameter of 15 nm, and the silica layer had a thickness of 20 nm.

«Production Example 2»

First, 1,000 g of zinc oxide fine particles (MZ-500, available from Tayca Corporation; having an average primary diameter of 25 nm) was taken by measurement, followed by the addition of water and mixing with stirring using a disper to give a slurry containing zinc oxide fine particles at a concentration of 100 g/L. To the resulting slurry was added 150 mL of an aqueous sodium silicate solution (already adjusted to a concentration of 200 g/L in terms of $SiO_2$) (3% by mass in terms of $SiO_2$, relative to the zinc oxide fine particles as a core material) while the slurry was stirred at room temperature. During the addition of an aqueous sodium silicate solution, hydrochloric acid was added to the slurry so that the pH of the slurry was maintained from 7 to 8, and cured for 15 minutes. Thereafter, silica-coated zinc oxide fine particles were obtained through the same operation as described in Production Example of silica-coated titanium oxide fine particles.

That is, the slurry after the curing was filtered and washed with water. Thus, a filtered cake was obtained, which contained 50% by mass of the zinc oxide fine particles, on the surface of each of the zinc oxide fine particles as the core material being formed, as a first layer, an aqueous silica layer at 3% by mass in terms of $SiO_2$, relative to the core material. Then, 2,060 g of this filtered cake without drying was taken by measurement, and to this filtered cake was added 5,000 g of isopropyl alcohol, followed by mixing with stirring using a disper to form a slurry. Further, to this slurry were added 1,000 g of deionized water and 50 g of aqueous ammonia, followed by further mixing with stirring, and the pH of the slurry was set to be from 10 to 11.

The resulting slurry was transferred to a horizontal bead mill (DYNO-MILL ECO-5, available from Willy A. Bachofen AG) at 150 mL/min. To this slurry were gradually added over 6 hours a mixed solution of 423 g of an oligomer of tetramethoxysilane having an average polymerization degree of about 5 (MKC Silicate MS51, available from Mitsubishi Chemical Corp.; having a molecular weight of 500 to 700 and a $SiO_2$ content of 52% by mass) and 212 g of isopropyl alcohol while the slurry was stirred within the horizontal bead mill to form, as a second layer, an aqueous silica layer at 22% by mass in terms of $SiO_2$, relative to the titanium oxide fine particles as the core material, on the aqueous silica layer as the above first layer. The addition speed at this time was 1.76 g/min. In the slurry after the addition of such an oligomer of tetramethoxysilane, neither remarkable thickening nor whitening was observed.

Then, the resulting slurry was transferred to a heating vacuum kneader, and water and isopropyl alcohol were distilled out by heating and pressure reducing. Thereafter, the residue was further heated to 150°C and then kept at the temperature for 2 hours to carry out curing. The powder obtained by such curing was pulverized with a JO mill (SK JET-O-MILL, available from Seishin Enterprise Co., Ltd.) to give silica-coated zinc oxide fine particles. The amount of silica coating of the silica-coated zinc oxide fine particles was 25% by mass in terms of $SiO_2$, relative to the zinc oxide fine particles as the core material. Then, 350 g of the resulting silica-coated zinc oxide fine particles and 650 g of deionized water were placed in a vessel, and the mixture was dispersed using a high-speed dispersing machine at 3,000 rpm for 5 minutes to give an aqueous dispersion of the silica-coated zinc oxide fine particles. This aqueous dispersion of the silica-coated zinc oxide fine particles was measured by a B-type viscometer (at 6 rpm at a solution temperature of 25°C), and it was found to have a viscosity of 30 mPa.s. Finally, 750 g of deionized water was added thereto to give an aqueous dispersion (ZS-1) of the silica-coated zinc oxide fine particles. This aqueous dispersion (ZS-1) of the silica-coated zinc oxide fine particles had a 20% non-volatile content. The zinc oxide fine particles had a primary particle diameter of 25 nm, and the silica layer had a thickness of 20 nm.

Next, the following will describe Production Examples of aqueous dispersions (ultraviolet screening agents for cosmetics) and powders of polymer-coated metal oxide fine particles produced by coating the surface of each of metal oxide fine particles with silica as a first layer and coating the outside of the first layer with a polymer as a second layer.

«Production Example 3»

To a 2-L reactor made of glass equipped with a stirrer, a dropping inlet, a nitrogen gas introducing tube, a thermometer, and a reflux condenser, 1,000 parts of the aqueous dispersion (TS-1) of the silica-coated titanium oxide fine particles and 10 parts of a 20% aqueous solution of Emarl 0 (available from Kao Corporation; sodium lauryl sulfate) were added under a nitrogen gas blow, followed by mixing, and then the mixture was heated to 80°C with stirring. Then, 20 parts of methyl methacrylate and 1.0 part of a 5% aqueous ammonium persulfate solution were added thereto. The mixture was kept with stirring for 5 hours to give an aqueous dispersion (PC-1) of the polymer-coated titanium oxide fine particles produced by coating the outside of each of the silica-coated titanium oxide fine particles with a polymer as a second layer.

The resulting aqueous dispersion (PC-1) of the polymer-coated titanium oxide fine particles had a total recovered amount of 1,031 g. When the aqueous dispersion (PC-1) of the polymer-coated titanium oxide fine particles was observed with a transmission electron microscope, it was confirmed that the surface of each of the silica-coated titanium oxide fine particles was coated with poly(methyl methacrylate) formed by polymerization.

Also, the fine particles contained in the aqueous dispersion (PC-1) of the polymer-coated titanium oxide fine particles were separated from the dispersion medium by centrifugation, and the resulting fine particles were washed with isopropyl alcohol and then vacuum dried (under a pressure of $1.33 \times 10^3$ Pa) at 50°C for 24 hours, to give a powder (PCP-1) of the polymer-coated titanium oxide fine particles produced by coating the outside of each of the silica-coated titanium oxide fine particles with a polymer as a second layer. The powder (PCP-1) of the polymer-coated titanium oxide fine particles had a number-average particle diameter of 80 nm, and when thermal mass loss was measured at the temperature-raising condition from 100°C to 500°C, mass loss of 9.7% was observed.

«Production Example 4»

To a 2-L reactor made of glass equipped with a stirrer, a dropping inlet, a nitrogen gas introducing inlet, a thermometer, and a reflux condenser, 1,000 parts of the aqueous dispersion (ZS-1) of the silica-coated zinc oxide fine particles and 15 parts of a 20% aqueous solution of Emulgen 109P (available from Kao Corporation; polyoxyethylene lauryl ether) were added under a nitrogen gas blow, followed by mixing, and then the mixture was heated to 80°C with stirring. Then, 40 parts of butyl methacrylate and 1.5 parts of a 5% aqueous ammonium persulfate solution were added thereto. The mixture was kept with stirring for 5 hours to give an aqueous dispersion (PC-2) of the polymer-coated zinc oxide fine particles produced by coating the outside of each of the silica-coated zinc oxide fine particle with a polymer as a second layer.

The resulting aqueous dispersion (PC-2) of the polymer-coated zinc oxide fine particles had a total recovered amount of 1,045 g. When the aqueous dispersion (PC-2) of the polymer-coated zinc oxide fine particles was observed with a transmission electron microscope, it was confirmed that the surface of each of the silica-coated titanium oxide fine particles was coated with poly(butyl methacrylate) formed by polymerization.

Also, the fine particles contained in the aqueous dispersion (PC-2) of the polymer-coated zinc oxide fine particles were separated from the dispersion medium by centrifugation, and the resulting fine particles were washed with isopropyl alcohol and then vacuum dried (under a pressure of $1.33 \times 10^3$ Pa) at 50°C for 24 hours, to give a powder (PCP-2) of the polymer-coated zinc oxide fine particles produced by coating the outside of each of the silica-coated zinc oxide fine particles with a polymer as a second layer. The powder (PCP-2) of the polymer-coated zinc oxide fine particles had a number-average particle diameter of 140 nm, and when thermal mass loss was measured at the temperature-raising condition from 100°C to 500°C, mass loss of 20.8% was observed.

«Production Example 5»

To a 2-L reactor made of glass equipped with a stirrer, a dropping inlet, a nitrogen gas introducing inlet, a thermometer, and a reflux condenser, 1,000 parts of the aqueous dispersion (TS-1) of the silica-coated titanium oxide fine particles, 9 parts of a 20% aqueous solution of Emarl 0 (available from Kao Corporation; sodium lauryl sulfate), and 6 parts of a 20% aqueous solution of Emarl 20CM (available from Kao Corporation; sodium polyoxyethylene alkyl ether sulfate) were added under a nitrogen gas blow, followed by mixing, and then the mixture was heated to 80°C with stirring. Then, 30 parts of methyl methacrylate, 30 parts of ethyl acrylate, and 2 parts of a 5% aqueous potassium persulfate solution were added thereto. The mixture was kept with stirring for 5 hours to give an aqueous dispersion (PC-3) of the polymer-coated titanium oxide fine particles produced by coating the outside of each of the silica-coated titanium oxide fine particles with a polymer as a second layer.

The resulting aqueous dispersion (PC-3) of the polymer-coated titanium oxide fine particles had a total recovered amount of 1,067 g. When the aqueous dispersion (PC-3) of the polymer-coated titanium oxide fine particles was observed with a transmission electron microscope, it was confirmed that the surface of each of the silica-coated titanium oxide fine particles was coated with a methyl methacrylate - ethyl acrylate copolymer formed by polymerization.

Also, the fine particles contained in the aqueous dispersion (PC-3) of the polymer-coated titanium oxide fine particles were separated from the dispersion medium by centrifugation, and the resulting fine particles were washed with isopropyl alcohol and then vacuum dried (under a pressure of 1.33 x 10³ Pa) at 50°C for 24 hours, to give a powder (PCP-3) of the polymer-coated titanium oxide fine particles produced by coating the outside of each of the silica-coated titanium oxide particles with a polymer as a second layer. The powder (PCP-3) of the polymer-coated titanium oxide fine particles had a number-average particle diameter of 100 nm, and when thermal mass loss was measured at the temperature-raising condition from 100°C to 500°C, mass loss of 28.9% was observed.

«Production Example 6»

To a 2-L reactor made of glass equipped with a stirrer, a dropping inlet, a nitrogen gas introducing inlet, a thermometer, and a reflux condenser, 1,000 parts of the aqueous dispersion (TS-1) of the silica-coated titanium oxide fine particles, 5 parts of a 20% aqueous solution of Emulgen 109P (available from Kao Corporation; sodium lauryl sulfate), and 5 parts of a 20% aqueous solution of Emarl 20CM (available from Kao Corporation; sodium polyoxyethylenealkyl ether sulfate) were added under a nitrogen gas blow, followed by mixing, and then the mixture was heated to 80°C with stirring. Then, 10 parts of KBE-503 (available from Shin-Etsu Chemical Co., Ltd.; γ-methacryloxypropyltriethoxysilane) was added dropwise thereto over 30 minutes with stirring, and after completion of the dropwise addition, the mixture was kept at 50°C for 5 hours.

Thereafter, the mixture was heated to 80°C, and 30 parts of methyl methacrylate and 2 parts of a 5% aqueous V-50 (available from Wako Pure Chemical Industries Ltd.; 2,2'-azobis(2-methyl-propionamidine) dihydrochloride) were added thereto. The mixture was kept with stirring for 5 hours to give an aqueous dispersion (PC-4) of the polymer-coated titanium oxide fine particles produced by coating the outside of each of the silica-coated titanium oxide fine particles with a polymer as a second layer.

The resulting aqueous dispersion (PC-4) of the polymer-coated titanium oxide fine particles had a total recovered amount of 1,046 g. When the aqueous dispersion (PC-4) of the polymer-coated titanium oxide fine particles was observed with a transmission electron microscope, it was confirmed that the surface of each of the silica-coated titanium oxide fine particles was coated with poly(methyl methacrylate) formed by polymerization.

Also, the fine particles contained in the aqueous dispersion (PC-4) of the polymer-coated titanium oxide fine particles were separated from the dispersion medium by centrifugation, and the resulting fine particles were washed with isopropyl alcohol and then vacuum dried (under a pressure of 1.33 x 10³ Pa) at 50°C for 24 hours, to give a powder (PCP-4) of the polymer-coated titanium oxide fine particles produced by coating the outside of each of the silica-coated titanium oxide fine particle with a polymer as a second layer. The powder (PCP-4) of the polymer-coated titanium oxide fine particles had a number-average particle diameter of 100 nm, and when thermal mass loss was measured at the temperature-raising condition from 100°C to 500°C, mass loss of 18.6% was observed.

«Production Example 7»

To a 2-L reactor made of glass equipped with a stirrer, a dropping inlet, a nitrogen gas introducing inlet, a thermometer, and a reflux condenser, 1,000 parts of the aqueous dispersion (ZS-1) of the silica-coated zinc oxide fine particles, 10 parts of a 20% aqueous solution of Emulgen 0 (available from Kao Corporation; sodium lauryl sulfate), and 5 parts of a 20% aqueous solution of Emulgen 109P (available from Kao Corporation; polyoxyethylene lauryl ether) were added under a nitrogen gas blow, followed by mixing, and then the mixture was heated to 80°C with stirring. Then, 5 parts of KBE-502 (available from Shin-Etsu Chemical Co., Ltd.; γ-methacryloxypropyldiethoxysilane) was added dropwise thereto over 30 minutes with stirring. After completion of the dropwise addition, the mixture was kept at 50°C for 5 hours.

Thereafter, the mixture was heated to 80°C, and 30 parts of methyl methacrylate, 10 parts of butyl acrylate, and 1 part of a 5% aqueous ammonium persulfate solution were added thereto. The mixture was kept with stirring for 5 hours to give a aqueous dispersion (PC-5) of the polymer-coated zinc oxide fine particles produced by coating the outside of each of the silica-coated zinc oxide fine particles with a polymer as a second layer.

The resulting aqueous dispersion (PC-5) of the polymer-coated zinc oxide fine particles had a total recovered amount of 1,055 g. When the aqueous dispersion (PC-5) of the polymer-coated zinc oxide fine particles was observed with a transmission electron microscope, it was confirmed that the surface of each of the silica-coated zinc oxide fine particle was coated with a methyl methacrylate - butyl acrylate copolymer formed by polymerization.

Also, the fine particles contained in the aqueous dispersion (PC-5) of the polymer-coated zinc oxide fine particles were separated from the dispersion medium by centrifugation, and the resulting fine particles were washed with isopropyl alcohol and then vacuum dried (under a pressure of 1.33 x 10³ Pa) at 50°C for 24 hours, to give a powder (PCP-5) of the polymer-coated zinc oxide fine particles produced by coating the outside of each of the silica-coated zinc oxide fine particles with a polymer as a second layer. The powder (PCP-5) of the polymer-coated zinc oxide fine particles had a number-average particle diameter of 150 nm, and when thermal mass loss was measured at the temperature-raising condition from 100°C to 500°C, mass loss of 21.3% was observed.

«Production Example 8»

To a 2-L reactor made of glass equipped with a stirrer, a dropping inlet, a nitrogen gas introducing inlet, a thermometer, and a reflux condenser, 1,000 parts of the aqueous dispersion (ZS-1) of the silica-coated zinc oxide fine particles and 10 parts of a 20% aqueous solution of Emulgen 109P (available from Kao Corporation; polyoxyethylene lauryl ether) were added under a nitrogen gas blow, followed by mixing, and then the mixture was heated to 80°C with stirring. Then, 5 parts of Prosil 9202 (available from Clariant Corporation; octyltriethoxysilane) was added dropwise thereto over 30 minutes with stirring. After completion of the dropwise addition, the mixture was kept at 50°C for 5 hours.
Thereafter, the mixture was heated to 80°C, and 30 parts of butyl methacrylate, 30 parts of butyl acrylate, and 1 part of a 5% aqueous potassium persulfate solution were added thereto. The mixture was kept with stirring for 5 hours to give an aqueous dispersion (PC-6) of the polymer-coated zinc oxide fine particles produced by coating the outside of each of the silica-coated zinc oxide fine particles with a polymer as a second layer.
The resulting aqueous dispersion (PC-6) of the polymer-coated zinc oxide fine particles had a total recovered amount of 1,066 g. When the aqueous dispersion (PC-6) of the polymer-coated zinc oxide fine particles was observed with a transmission electron microscope, it was confirmed that the surface of each of the silica-coated zinc oxide fine particles was coated with a butyl methacrylate - butyl acrylate copolymer formed by polymerization.
Also, the fine particles contained in the aqueous dispersion (PC-6) of the polymer-coated zinc oxide fine particles were separated from the dispersion medium by centrifugation, and the resulting fine particles were washed with isopropyl alcohol and then vacuum dried (under a pressure of $1.33 \times 10^3$ Pa) at 50°C for 24 hours to give a powder (PCP-6) of the polymer-coated zinc oxide fine particles produced by coating the outside of each of the silica-coated zinc oxide fine particles with a polymer as a second layer. The powder (PCP-6) of the polymer-coated zinc oxide fine particles had a number-average particle diameter of 180 nm, and when thermal mass loss was measured at the temperature-raising condition from 100°C to 500°C, mass loss of 31.1% was observed.

«Production Example 9»

To a 2-L reactor made of glass equipped with a stirrer, a dropping inlet, a nitrogen gas introducing inlet, a thermometer, and a reflux condenser, 1,000 parts of the aqueous dispersion (TS-1) of the silica-coated titanium oxide fine particles and 15 parts of a 20% aqueous solution of Emarl 20CM (available from Kao Corporation; sodium polyoxyethylene alkyl ether sulfate) were added under a nitrogen gas blow, followed by mixing, and then the mixture was heated to 80°C with stirring. Then, 3 parts of KBE-503 (available from Shin-Etsu Chemical Co., Ltd.; γ-methacryloxypropyltriethoxysilane) and 3 parts of KBE-502 (available from Shin-Etsu Chemical Co., Ltd.; γ-methacryloxypropyldiethoxysilane) were added dropwise thereto over 30 minutes with stirring, and after completion of the dropwise addition, the mixture was kept at 50°C for 5 hours.
Thereafter, the mixture was heated to 80°C, and 20 parts of methyl methacrylate, 20 parts of butyl methacrylate, 20 parts of butyl acrylate; and 1.2 parts of a 5% aqueous ammonium persulfate solution were added thereto. The mixture was kept with stirring for 5 hours to give an aqueous dispersion (PC-7) of the polymer-coated titanium oxide fine particles produced by coating the outside of each of the silica-coated titanium oxide fine particles with a polymer as a second layer.
The resulting aqueous dispersion (PC-7) of the polymer-coated titanium oxide fine particles had a total recovered amount of 1,060 g. When the aqueous dispersion (PC-7) of the polymer-coated titanium oxide fine particles was observed with a transmission electron microscope, it was confirmed that the surface of each of the silica-coated titanium oxide fine particles was coated with a methyl methacrylate - butyl methacrylate - butyl acrylate copolymer formed by polymerization.
Also, the fine particles contained in the aqueous dispersion (PC-7) of the polymer-coated titanium oxide fine particles were separated from the dispersion medium by centrifugation, and the resulting fine particles were washed with isopropyl alcohol and then vacuum dried (under a pressure of $1.33 \times 10^3$ Pa) at 50°C for 24 hours to give a powder (PCP-7) of the polymer-coated titanium oxide fine particles produced by coating the outside of each of the silica-coated titanium oxide particles with a polymer as a second layer. The powder (PCP-7) of the polymer-coated titanium oxide fine particles had a number-average particle diameter of 140 nm, and when thermal mass loss was measured at the temperature-raising condition from 100°C to 500°C, mass loss of 32.0% was observed.

«Production Example 10»

To a 2-L reactor made of glass equipped with a stirrer, a dropping inlet, a nitrogen gas introducing inlet, a thermometer, and a reflux condenser, 1,000 parts of the aqueous dispersion (TS-1) of the silica-coated titanium oxide fine particles and 20 parts of a 20% aqueous solution of Emarl 20CM (available from Kao Corporation; sodium polyoxyethylene alkyl ether sulfate) were added under a nitrogen gas blow, followed by mixing, and then the mixture was heated to 80°C with stirring. Then, 8 parts of Prosil 9202 (available from Clariant Corporation; octyltriethoxysilane) was added dropwise thereto over 30 minutes with stirring. After completion of the dropwise addition, the mixture was kept at 50°C for 5 hours.

Thereafter, the mixture was heated to 80°C, and 40 parts of methyl methacrylate, 40 parts of butyl methacrylate, 20 parts of butyl acrylate, and 2 parts of a 5% aqueous ammonium persulfate solution were added thereto. The mixture was kept with stirring for 5 hours to give an aqueous dispersion (PC-8) of the polymer-coated titanium oxide fine particles produced by coating the outside of each of the silica-coated titanium oxide fine particles with a polymer as a second layer.

The resulting aqueous dispersion (PC-8) of the polymer-coated titanium oxide fine particles had a total recovered amount of 1,100 g. When the aqueous dispersion (PC-8) of the polymer-coated titanium oxide fine particles was observed with a transmission electron microscope, it was confirmed that the surface of each of the silica-coated titanium oxide fine particles was coated with a methyl methacrylate - butyl methacrylate - butyl acrylate copolymer formed by polymerization.

Also, the fine particles contained in the aqueous dispersion (PC-8) of the polymer-coated titanium oxide fine particles were separated from the dispersion medium by centrifugation, and the resulting fine particles were washed with isopropyl alcohol and then vacuum dried (under a pressure of $1.33 \times 10^3$ Pa) at 50°C for 24 hours to give a powder (PCP-8) of the polymer-coated titanium oxide fine particles produced by coating the outside of each of the silica-coated titanium oxide fine particles with a polymer as a second layer. The powder (PCP-8) of the polymer-coated titanium oxide fine particles had a number-average particle diameter of 150 nm, and when thermal mass loss was measured at the temperature-raising condition from 100°C to 500°C, mass loss of 52.5% was observed.

Next, the following will describe the production and evaluation of cosmetics using the aqueous dispersions (ultraviolet screening agents for cosmetics) of the polymer-coated metal oxide fine particles obtained above.

«Examples 1 to 8 and Comparative Examples 1 to 2»

First, 4 parts of squalane (Fitoderm, available from Iwase Cosfa Co., Ltd.) and 0.4 parts of polyglyceryl-6 stearate (NIKKOL Hexaglyn 1-SV, available from Nikko Chemicals Co., Ltd.) were mixed with stirring using a disper to give composition A. Also, 15 parts of purified water, 5 parts of butylene glycol (1,3-BG, available from Kyowa Hakko Kogyo Co., Ltd.), 0.2 parts of methylparaben (Mekkins M, available from Ueno Fine Chemicals Industry, Ltd.), and 0.6 parts of polysorbate 60 (Rheodol TW-S 120V, available from Kao Corporation; polyoxyethylenesorbitan monostearate) were mixed with stirring using a disper to give composition B. Further, 40.3 parts of purified water, 0.3 parts of carbomer (Carbopol 940, available from Noveon, Inc.; carboxyvinyl polymer), 0.05 parts of xanthan gum (Neosoft XZ, available from Taiyo Kagaku Co., Ltd.), 10 parts of glycerin (concentrated glycerin for cosmetics, available from Kao Corporation; having a glycerin concentration of not lower than 98.5%), and 0.09 parts of sodium hydroxide were mixed with stirring using a disper to give composition C.

Then, composition A was added to composition B, and the mixture was emulsified, after which composition C was added to the mixture, followed by stirring. Finally, 24 parts of the aqueous dispersion (PC-1) of the polymer-coated titanium oxide fine particles, a nonvolatile content of which had been adjusted to 20% by the addition of purified water, was added as an ultraviolet screening agent for cosmetics, and the mixture was stirred to become uniform. Thus, gel cosmetic (BC-1) was obtained.

Also, gel cosmetics (BC-2) to (BC-8) were obtained in the same manner as describe above, except that the aqueous dispersion (PC-2), (PC-5), or (PC-6) of the polymer-coated zinc oxide fine particles, or the aqueous dispersion (PC-3), (PC-4), (PC-7), or (PC-8) of the polymer-coated titanium oxide fine particles, was used as an ultraviolet screening agent for cosmetics, in place of the aqueous dispersion (PC-1) of the polymer-coated titanium oxide fine particles.

Further, gel cosmetics for comparison (BN-1) and (BN-2) were obtained in the same manner as described above, except that the aqueous dispersion (TS-1) of the silica-coated titanium oxide fine particles or the aqueous dispersion (ZS-1) of the silica-coated zinc oxide fine particles was used as an ultraviolet screening agent for cosmetics, in place of the aqueous dispersion (PC-1) of the polymer-coated titanium oxide fine particles.

«Examples 9 to 16 and Comparative Examples 3 to 4»

First, 5.0 parts of titanium oxide fine particles (MT-100TV, available from Tayca Corporation; having an average primary particle diameter of 15 nm), 20.0 parts of (C12 to C15) alkyl benzoate (FINSOLV TN, available from Finetex Inc.), 2.5 parts of self-emulsifiable glyceryl stearate (NIKKOL MGS-BSEV, available from Nikko Chemicals Co., Ltd.), 2.0 parts of stearic acid (purified stearic acid 450V, available from Kao Corporation), and 1.0 part of cetanol (Conol 300C, available from New Japan Chemical Co., Ltd.) were mixed under heating at 80°C and stirred to become uniform. Thus, composition D was obtained.

Then, to 0.2 parts of xanthan gum (Neosoft XZ, available from Taiyo Kagaku Co., Ltd.), 1.0 part of TEA (triethanolamine), 6.5 parts of butylene glycol (1,3-BG, available from Kyowa Hakko Kogyo Co., Ltd.), 0.5 parts of polysorbate 65 (NIKKOL TS-30V, available from Nikko Chemicals Co., Ltd.), and 36.3 parts of deionized water, 25 parts of the aqueous dispersion (PC-1) of the polymer-coated titanium oxide fine particles, a nonvolatile content of which had been adjusted to 20% by the addition of purified water, was added as an ultraviolet screening agent for cosmetics, and the mixture was mixed under heating at 80°C and stirred to become uniform. Then, composition D was added thereto, and the mixture was emulsified and cooled to give O/W cream cosmetic (BC-9).

Also, O/W cream cosmetics (BC-10) to (BC-16) were obtained in the same manner as described above, except that the aqueous dispersion (PC-2), (PC-5), or (PC-6) of the polymer-coated zinc oxide fine particles, or the aqueous dispersion (PC-3), (PC-4), (PC-7), or (PC-8) of the polymer-coated titanium oxide fine particles, was used as an ultraviolet screening agent for cosmetics, in place of the aqueous dispersion (PC-1) of the polymer-coated titanium oxide fine particles.

Further, O/W cream cosmetics for comparison (BN-3) and (BN-4) were obtained in the same manner as described as above, except that the aqueous dispersion (TS-1) of the silica-coated titanium oxide fine particles or the aqueous dispersion (ZS-1) of the silica-coated zinc oxide fine particles was used as an ultraviolet screening agent for cosmetics, in place of the aqueous dispersion (PC-1) of the polymer-coated titanium oxide particles.

«Examples 17 to 24 and Comparative Examples 5 to 6»

First, 49.7 parts of (C12 to C15) alkyl benzoate (FINSOLV TN, available from Finetex Inc.), 2.5 parts of sorbitan sesquiiso-stearate (EMALEX SPIS-150, available from Nihon Emulsion Co., Ltd.), 0.8 parts of polyglyceryl-10 stearate (NIKKOL Decaglyn 1-0V, available from Nikko Chemicals Co., Ltd.), and 5.0 parts of titanium oxide fine particles (MT-100Z, available from Tayca Corporation; having an average primary diameter of 15 nm) were mixed under heating at 60°C and stirred to become uniform. Thus, composition E was obtained.

Then, to 20.0 parts of deionized water and 7.0 parts of butylene glycol (1,3-BG, available from Kyowa Hakko Kogyo Co., Ltd.), 15 parts of the aqueous dispersion (PC-1) of the polymer-coated titanium oxide fine particles, a nonvolatile content of which had been adjusted to 20% by the addition of purified water, was added as an ultraviolet screening agent for cosmetics and stirred to become uniform. Then, composition E was added thereto, and the mixture was mixed under heating art 60°C and stirred to become uniform. Thus, W/O liquid sun screening milk cosmetic (BC-17) was obtained.

Also, W/O liquid sun screening milk cosmetics (BC-18) to (BC-24) were obtained in the same manner as described above, except that the aqueous dispersion (PC-2), (PC-5), or (PC-6) of the polymer-coated zinc oxide fine particles, or the aqueous dispersion (PC-3), (PC-4), (PC-7), or (PC-8) of the polymer-coated titanium oxide fine particles, was used as an ultraviolet screening agent for cosmetics, in place of the aqueous dispersion (PC-1) of the polymer-coated titanium oxide fine particles.

Further, W/O liquid sun screening milk cosmetics for comparison (BN-5) and (BN-6) were obtained in the same manner as described above, except that the aqueous dispersion (TS-1) of the silica-coated titanium oxide fine particles or the aqueous dispersion (ZS-1) of the silica-coated zinc oxide fine particles was used as an ultraviolet screening agent for cosmetics, in place of the aqueous dispersion (PC-1) of the polymer-coated titanium oxide fine particles.

«Examples 25 to 32 and Comparative Examples 7 to 8»

First, 15.0 parts of deionized water, 5.0 parts of butylene glycol (1,3-BG, available from Kyowa Hakko Kogyo Co., Ltd.), 0.2 parts of methylparaben (Mekkins M, available from Ueno Fine Chemicals Industry, Ltd.), and 0.6 parts of polysorbate 60 (Rheodol TW-S 120V, available from Kao Corporation; polyoxyethylenesorbitan fatty acid ester) were mixed with stirring at 60°C to become uniform. Thus, composition F was obtained. Also, 4.0 parts of octyl methoxycinnamate (PARSOL MCX, available from DHL), and 0.4 parts of polyglyceryl-6 stearate (NIKKOL Hexaglyn, 1-SV available from Nikko Chemicals Co., Ltd.) were mixed with stirring to become uniform. Thus, composition G was obtained. Further, 40.3 parts of deionized water, 0.3 parts of carbomer (Carbopol 940, available from Noveon, Inc.; carboxyvinyl polymer), 0.05 parts of xanthan gum (Neosoft XZ, available from Taiyo Kagaku Co., Ltd.), 10 parts of glycerin (concentrated glycerin for cosmetics, available from Kao Corporation; having a glycerin concentration of not lower than 98.5%), and 0.09 parts of sodium hydroxide were mixed with stirring to become uniform. Thus, composition H was obtained.

Then, composition G was added to composition F while heating to 60°C, and the mixture was stirred to become uniform, after which composition H was added thereto, followed by stirring. Further, 24 parts of the aqueous dispersion (PC-1) of the polymer-coated titanium oxide fine particles, a nonvolatile content of which had been adjusted to 20% by the addition of purified water, was added as an ultraviolet screening agent for cosmetics, and the mixture was stirred to give gel cosmetic (BC-25).

Also, gel cosmetics (BC-25) to (BC-32) were obtained in the same manner as described above, except that the aqueous dispersion (PC-2), (PC-5), or (PC-6) of the polymer-coated zinc oxide fine particles, or the aqueous dispersion (PC-3), (PC-4), (PC-7), or (PC-8) of the polymer-coated titanium oxide fine particles, was used as an ultraviolet screening agent for cosmetics, in place of the aqueous dispersion (PC-1) of the polymer-coated titanium oxide fine particles.

Further, gel cosmetics for comparison (BN-7) and (BN-8) were obtained in the same manner as described above, except that the aqueous dispersion (TS-1) of the silica-coated titanium oxide fine particles or the aqueous dispersion (ZS-1) of the silica-coated zinc oxide fine particles was used as an ultraviolet screening agent for cosmetics, in place of the aqueous dispersion (PC-1) of the polymer-coated titanium oxide fine particles.

The resulting cosmetics (BC-1) to (BC-32) and cosmetics for comparison (BN-1) to (BN-8) were tested for the characteristics below and their performances were evaluated. The results are shown in Tables 1 to 4.

<Transparency>

A cosmetic was coated on a transparent glass plate using an applicator in such a manner that the wet film thickness became 10 $\mu$m, and then its transparency was visually evaluated on the following criteria.

⊙ : extremely transparent;
○ : transparent;
Δ : slightly transparent; and
x: not transparent at all.

<Storage stability>

A cosmetic was allowed to stand at 50°C for 30 days, and then testing as in the transparency above was carried out, and its transparency was visually evaluated on the following criteria.

⊙ : extremely transparent and excellent in storage stability;
○ : transparent and good in storage stability;
Δ : slightly transparent and slightly good in storage stability; and
x: not transparent at all and poor in storage stability.

<Dispersibility>

A cosmetic was placed on a slide glass plate in an appropriate amount and observed under a digital microscope (VHX-500, available from Keyence Corporation) to count the number of particles having a longest diameter of 5 $\mu$m or greater per 0.0025 mm$^2$ and evaluate the dispersibility on the following criteria.

5 points: coarse particles having a longest diameter of 5 $\mu$m or greater are not observed;
4 points: the number of coarse particles having a longest diameter of 5 $\mu$m or greater is from 1 to 3;
3 points: the number of coarse particles having a longest diameter of 5 $\mu$m or greater is from 4 to 10;
2 points: more than 10 particles having a longest diameter of 5 $\mu$m or greater are observed;
1 point: almost all the fine particles aggregate.

<Feel to the skin>

Thirty women of from 20 to 50 years old were subjected to the application of a cosmetic to their skins, and on the basis of the number of women who answered excellent in a feel, the feel to the skin was evaluated on the following criteria.

⊙ : 24 or more women;
○ : from not less than 18 to less than 24 women;
A: from not less than 12 to less than 18 women; and
x: less than 12 women.

TABLE 1

| | Cosmetics | Ultraviolet screening agents for cosmetics | Transparency | Storage stability | Dispersibility | Feel to the skin |
|---|---|---|---|---|---|---|
| Example 1 | BC-1 | PC-1 | Δ | Δ | 4 points | ○ |
| Example 2 | BC-2 | PC-2 | ○ | ○ | 3 points | Δ |
| Example 3 | BC-3 | PC-3 | Δ | Δ | 4 points | ○ |
| Example 4 | BC-4 | PC-4 | ○ | ○ | 5 points | ⊙ |
| Example 5 | BC-5 | PC-5 | ⊙ | ⊙ | 5 points | ⊙ |
| Example 6 | BC-6 | PC-6 | ⊙ | ⊙ | 5 points | ⊙ |
| Example 7 | BC-7 | PC-7 | ○ | ○ | 5 points | ⊙ |
| Example 8 | BC-8 | PC-8 | ○ | ○ | 5 points | ⊙ |
| Comp. Ex. 1 | BN-1 | TS-1 | x | x | 1 point | x |
| Comp. Ex. 2 | BN-2 | ZS-1 | x | x | 1 point | x |

TABLE 2

| | Cosmetics | Ultraviolet screening agents for cosmetics | Transparency | Storage stability | Dispersibility | Feel to the skin |
|---|---|---|---|---|---|---|
| Example 9 | BC-9 | PC-1 | ○ | ○ | 5 points | ○ |
| Example 10 | BC-10 | PC-2 | ⊙ | ○ | 5 points | ○ |
| Example 11 | BC-11 | PC-3 | ○ | ○ | 5 points | ⊙ |
| Example 12 | BC-12 | PC-4 | ○ | ○ | 5 points | ○ |
| Example 13 | BC-13 | PC-5 | ⊙ | ⊙ | 5 points | ⊙ |
| Example 14 | BC-14 | PC-6 | ⊙ | ⊙ | 5 points | ⊙ |
| Example 15 | BC-15 | PC-7 | ○ | ○ | 5 points | ○ |
| Example 16 | BC-16 | PC-8 | ○ | ○ | 5 points | ○ |
| Comp. Ex. 3 | BN-3 | TS-1 | x | x | 2 points | x |
| Comp. Ex. 4 | BN-4 | ZS-1 | x | x | 2 points | x |

TABLE 3

| | Cosmetics | Ultraviolet screening agents for cosmetics | Transparency | Storage stability | Dispersibility | Feel to the skin |
|---|---|---|---|---|---|---|
| Example 17 | BC-17 | PC-1 | ⊙ | ⊙ | 5 points | ○ |
| Example 18 | BC-18 | PC-2 | ⊙ | ⊙ | 5 points | ○ |
| Example 19 | BC-19 | PC-3 | ⊙ | ⊙ | 5 points | ⊙ |
| Example 20 | BC-20 | PC-4 | ⊙ | ⊙ | 5 points | ○ |
| Example 21 | BC-21 | PC-5 | ⊙ | ⊙ | 5 points | ⊙ |

(continued)

|  | Cosmetics | Ultraviolet screening agents for cosmetics | Transparency | Storage stability | Dispersibility | Feel to the skin |
|---|---|---|---|---|---|---|
| Example 22 | BC-22 | PC-6 | ⊙ | ⊙ | 5 points | ⊙ |
| Example 23 | BC-23 | PC-7 | ⊙ | ⊙ | 5 points | ○ |
| Example 24 | BC-24 | PC-8 | ⊙ | ⊙ | 5 points | ○ |
| Comp. Ex. 5 | BN-5 | TS-1 | x | x | 2 points | Δ |
| Comp. Ex. 6 | BN-6 | ZS-1 | Δ | x | 2 points | x |

TABLE 4

|  | Cosmetics | Ultraviolet screening agents for cosmetics | Transparency | Storage stability | Dispersibility | Feel to the skin |
|---|---|---|---|---|---|---|
| Example 25 | BC-25 | PC-1 | ○ | ○ | 4 points | ○ |
| Example 26 | BC-26 | PC-2 | ○ | ○ | 3 points | ○ |
| Example 27 | BC-27 | PC-3 | ○ | ○ | 4 points | ○ |
| Example 28 | BC-28 | PC-4 | ○ | ○ | 5 points | ○ |
| Example 29 | BC-29 | PC-5 | ⊙ | ⊙ | 5 points | ⊙ |
| Example 30 | BC-30 | PC-6 | ⊙ | ⊙ | 5 points | ⊙ |
| Example 31 | BC-31 | PC-7 | ○ | ○ | 5 points | ⊙ |
| Example 32 | BC-32 | PC-8 | ○ | ○ | 5 points | ⊙ |
| Comp. Ex. 7 | BN-7 | TS-1 | x | x | 1 point | x |
| Comp. Ex. 8 | BN-8 | ZS-1 | x | x | 1 point | x |

AS can be seen from Tables 1 to 4, the gel cosmetics of Examples 1 to 8, the O/W cream cosmetics of Examples 9 to 16, the W/O liquid sun screening milk cosmetics of Examples 17 to 24, and the gel cosmetics of Examples 25 to 32, each of which contains, as an ultraviolet screening agent for cosmetics, a dispersion of polymer-coated metal oxide fine particles produced by coating, with silica as a first layer, the surface of each of metal oxide fine particles, and coating the outside of the first layer with a polymer as a second layer, are all transparent and excellent in storage stability, dispersibility, and a feel to the skin.

In contrast, the gel cosmetics for comparison of Comparative Examples 1 and 2, the O/W cream cosmetics for comparison of Comparative Examples 3 and 4, the W/O liquid sun screening milk cosmetics for comparison of Examples 5 and 6, and the gel cosmetics for comparison of Comparative Examples 7 and 8, each of which contains, as an ultraviolet screening agent for cosmetics, a dispersion of polymer-coated metal oxide fine particles produced by coating, with silica as a first layer, the surface of each of metal oxide fine particles, and not coating the outside of the first layer with a polymer as a second layer, are not transparent and inferior in storage stability, dispersability, and a feel to the skin.

Thus, when a dispersion and/or a powder of polymer-coated metal oxide fine particles produced by coating, with silica as a first layer, the surface of each of metal oxide fine particles, and coating the outside of the first layer with a specific polymer as a second layer, is used as an ultraviolet screening agent for cosmetics, such an ultraviolet screening agent for cosmetics has excellent dispersibility and excellent storage stability while maintaining high transparency and excellent ultraviolet screening ability and gives cosmetics exhibiting a remarkably improved feel to the skin when added to the cosmetics.

The ultraviolet screening agent for cosmetics according to the present invention has excellent dispersibility and excellent storage stability while maintaining high transparency and excellent ultraviolet screening ability and gives cosmetics exhibiting a remarkably improved feel to the skin when added to the cosmetics. Therefore, the ultraviolet screening agent for cosmetics according to the present invention makes a great contribution in the fields of cosmetics and cosmetic bases.

## Claims

1. An ultraviolet screening agent for cosmetics, comprising polymer-coated metal oxide fine particles produced by coating, with silica as a first layer, a surface of each of at least one kind of metal oxide fine particles selected from a group consisting of zinc oxide fine particles, titanium oxide fine particles, cerium oxide fine particles, zirconium oxide fine particles, and iron oxide fine particles, a primary particle diameter of which fine particles is not smaller than 1 nm and not greater than 100 nm, and coating an outside of the first layer with a polymer as a second layer, wherein the polymer coating is chemically bonded to the surface of each of the silica-coated metal oxide fine particles and wherein the polymer includes (meth)acrylic-type polymers, styrene-type polymers, vinyl acetate-type polymers, vinyl chloride-type polymers, vinylidene-type polymers, and their copolymers used alone or two or more kinds of these polymers may be used in combination.

2. The ultraviolet screening agent for cosmetics according to claim 1, wherein the polymer-coated metal oxide fine particles are in a form of a dispersion and/or a powder.

3. A cosmetic comprising an ultraviolet screening agent for cosmetics according to claim 1 or 2.

4. The cosmetic according to claim 3, further comprising a carboxyvinyl polymer as a hydrophilic thickener or an emulsion stabilizer.

## Patentansprüche

1. Ultraviolett-Schutzmittel für Kosmetika, umfassend polymerbeschichtete Metalloxid-Feinpartikel, hergestellt durch das Beschichten einer Oberfläche von jedem der mindestens einen Art von Metalloxid-Feinpartikel, ausgewählt aus einer Gruppe, bestehend aus Zinkoxid-Feinpartikel, Titanoxid-Feinpartikel, Ceroxid-Feinpartikel, Zirkoniumoxid-Feinpartikel und Eisenoxid-Feinpartikel, wobei ein primärer Teilchendurchmesser dieser Feinpartikel nicht kleiner als 1 nm und nicht größer als 100 nm ist, mit Siliciumoxid als eine erste Schicht und das Beschichten einer Außenseite der ersten Schicht mit einem Polymer als eine zweite Schicht, wobei die Polymerbeschichtung chemisch an die Oberfläche von jedem der siliciumoxidbeschichteten Metalloxid-Feinpartikel gebunden ist und wobei das Polymer Polymere des (Meth)acryltyps, Polymere des Styroltyps, Polymere des Vinylacetattyps, Polymere des Vinylchloridtyps, Polymere des Vinylidentyps und deren Copolymere einschließt, die alleine oder zwei oder mehr Arten dieser Polymere in Kombination verwendet werden können.

2. Ultraviolett-Schutzmittel für Kosmetika nach Anspruch 1, wobei die polymerbeschichteten Metalloxid-Feinpartikel in Form einer Dispersion und/oder eines Pulvers vorliegen.

3. Kosmetikum, umfassend ein Ultraviolett-Schutzmittel für Kosmetika nach Anspruch 1 oder 2.

4. Kosmetikum nach Anspruch 3, weiter umfassend ein Carboxyvinylpolymer als ein hydrophiles Verdickungsmittel oder einen Emulsionsstabilisator.

## Revendications

1. Un agent filtrant les ultraviolets pour des produits cosmétiques, comprenant des particules fines d'oxyde de métal enrobées d'un polymère produites par enrobage, avec du silice en guise de première couche, une surface de chacun d'au moins un type de particules fines d'oxyde de métal sélectionné parmi un groupe consistant en des particules fines d'oxyde de zinc, des particules fines d'oxyde de titane, des particules fines d'oxyde de cérium, des particules fines d'oxyde de zirconium et des particules fines d'oxyde de fer, un diamètre de particule primaire desdites particules fines n'étant pas inférieur à 1 nm et pas supérieur à 100 nm, et l'enrobage d'un extérieur de la première couche avec un polymère en tant que seconde couche, dans lequel l'enrobage de polymère est chimiquement lié à la surface de chacune des particules fines d'oxyde de métal enrobées de silice et dans lequel le polymère comprend des polymères de type (méth)acrylique, des polymères de type styrène, des polymères de type acétate de vinyle, des polymères de type chlorure de vinyle, des polymères de type vinylidène, et leurs copolymères utilisés seuls ou deux ou plusieurs de ces polymères peuvent être utilisés en combinaison.

2. L'agent filtrant les ultraviolets pour des produits cosmétiques selon la revendication 1, dans lequel les particules fines d'oxyde de métal enrobées de polymère sont dans la forme d'une dispersion et/ou d'une poudre.

3. Un cosmétique comprenant un agent filtrant les ultraviolets pour des produits cosmétiques selon la revendication 1 ou 2

4. Le cosmétique selon la revendication 3, comprenant par ailleurs un polymère de carboxyvinyle en tant qu'épaississant hydrophile ou un stabilisateur d'émulsion.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI3183620 B
- JP HEI11302015 B
- JP 2001058821 A
- WO 01093812 A
- DE 102004041536 A1
- DE 102005010803 A1